# EUROPEAN PATENT SPECIFICATION

(11) **EP 3 259 358 B1**
(45) Date of publication and mention of the grant of the patent: **19.06.2024**
(21) Application number: 16710358.9
(22) Date of filing: 19.02.2016
(51) Int. Cl.: C12N 15/68

(54) **ENHANCED PROTEIN EXPRESSION**
ERHÖHTE PROTEINEXPRESSION
EXPRESSION AMÉLIORÉE DE PROTÉINE

(30) Priority: 19.02.2015 US 201562118382 P
(43) Date of publication of application: 27.12.2017
(73) Proprietor: Danisco US Inc., Palo Alto, California 94304 (US)
(72) Inventor: BONGIORNI, Cristina, Palo Alto, California 94304 (US); DELANGE, Dennis, Palo Alto, California 94304 (US); ENGLAND, George, Palo Alto, California 94304 (US); KOLKMAN, Marc, Palo Alto, California 94304 (US); LEEFLANG, Chris, Palo Alto, California 94304 (US)
(74) Representative: Mewburn Ellis LLP
(86) International application number: PCT/US2016/018598
(87) International publication number: WO 2016/134213

(56) References cited:
- WO-A1-2010/005525
- WO-A1-2010/144283
- WO-A1-2012/114234
- WO-A2-2008/148575
- WO-A2-2014/004638
- Anonymous: "Bacillus stearothermophilus alpha amylase (ami) gene, complete cds - Nucleotide - NCBI", , 25 November 1997 (1997-11-25), XP055806591, Retrieved from the Internet: URL:https://www.ncbi.nlm.nih.gov/nuccore/A F032864.1 [retrieved on 2021-05-21]

## Description

### CROSS-REFERENCE TO RELATED APPLICATIONS

The present application claims priority to currently pending U.S. Provisional Patent Application Serial No. 62/118,382, filed February 19, 2015.

### REFERENCE TO THE SEQUENCE LISTING

The contents of the electronic submission of the text file Sequence Listing, named "NB40178WOPCT-Sequence-Listing.txt" was created on February 17, 2015 and is 27 KB in size.

### FIELD OF THE INVENTION

The present invention relates in general to bacterial cells having genetic alterations that results in increased expression of a protein of interest and methods of making and using such cells. Aspects of the present invention include Gram-positive microorganisms, such as *Bacillus* species, having a genetic alteration that results in enhanced expression of a protein of interest.

### BACKGROUND OF THE INVENTION

Genetic engineering has allowed the improvement of microorganisms used as industrial bioreactors, cell factories and in food fermentations. Gram-positive organisms, including a number of *Bacillus* species, are used to produce a large number of useful proteins and metabolites (see, e.g., Zukowski, "Production of commercially valuable products," In: Doi and McGlouglin (eds.) Biology of Bacilli: Applications to Industry, Butterworth-Heinemann, Stoneham. Mass pp 311-337 [1992]). Common *Bacillus* species used in industry include, but are not limited to, *B. licheniformis, B. amyloliquefaciens* and *B*. *subtilis.* In addition to their use in general industrial settings (e. g., enzymes for detergents, biomass fuel production amongst other things); because of their GRAS (generally recognized as safe) status, strains of these *Bacillus* species are natural candidates for the production of proteins utilized in the food and pharmaceutical industries. Examples of proteins produced in Gram-positive organisms include, but are not limited to, enzymes, e.g., amylases, neutral proteases, alkaline (or serine) proteases, and pullulanases.

In spite of advances in the understanding of production of proteins in bacterial host cells, there remains a need to develop new recombinant strains that express increased levels of a protein of interest.

WO/2010/144283 discloses a Bacilllus strain for increased protein production. WO/2008/148575 relates to increased production of a target product via stabilisation of mRNA. WO/2010/005525 discloses polymers of isoprene from renewable sources. WO/2012/114234 A1 relates to industrial processing of plant-derived food and feed products.

### SUMMARY OF THE INVENTION

In certain embodiments, the present invention is directed to recombinant (i.e., genetically modified) bacterial (host) cells, and methods thereof for increased expression of a gene of interest encoding a protein of interest. In certain other embodiments, the invention is directed to the increased production of one or more proteins of interest in a recombinant (modified) bacterial host cell of the disclosure. In yet other embodiments, the invention is directed to methods of modifying bacterial host cells for expressing and/or producing one or more proteins of interest. In certain other embodiments, the invention is directed to the surprising and unexpected results set forth herein, demonstrating that the use of certain strong 5' UTRs in operable combination with a gene of interest results in higher expression of the protein of interest.

In a first aspect, the invention provides a DNA molecule comprising, in a 5' to 3' direction, a 5' untranslated region (5' UTR) nucleic acid sequence operably linked to a nucleic acid sequence encoding a gene of interest (GOI), wherein the 5' UTR is derived from a *Bacillus* species (spp.) aprE protease gene, wherein the 5' UTR is heterologous to the GOI in which the 5' UTR is operably linked, and wherein the 5' UTR comprises the sequence of SEQ ID NO: 9.

In a second aspect, the invention provides an expression cassette or vector comprising a DNA molecule of the first aspect.

In a third aspect, the invention provides a host cell comprising an expression cassette or vector of the second aspect, or a DNA molecule of the first aspect.

In some embodiments, the host cell is a *Bacillus* species. In some embodiments, the Bacillus species is selected from the group consisting of *B*. *licheniformis, B. lentus, B. subtilis, B. amyloliquefaciens, B. brevis, B. stearothermophilus, B. alkalophilus, B. coagulans, B. circulans, B. pumilus, B. lautus, B. clausii, B. megaterium,* and *B*. *thuringiensis.* In another aspect, the *Bacillus sp.* strain is a *B*. *subtilis* strain. In another aspect, the *Bacillus sp.* strain is a *B*. *licheniformis* strain.

In some embodiments, the gene of interest codes for an enzyme. In some embodiments, the enzyme is an amylase or pullinase. In some embodiments, the gene of interest codes for a wild-type Bacillus spp. enzyme or a variant Bacillus app. enzyme. In some embodiments, the enzyme is selected from the group consisting of acyl transferases, alpha-amylases, β-amylases, alpha-galactosidases, arabinosidases, aryl esterases, beta-galactosidases, carrageenases, catalases, cellobiohydrolases, cellulases, chondroitinases, cutinases, endo-beta-1,4-glucanases, endo-p-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, perhydrolases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, and xylosidases.

In a fourth aspect, the invention provides a DNA molecule comprising a sequence having 90% sequence identity to SEQ ID NO: 15-when determined using the Clustal alignment tool, wherein the DNA molecule comprises in the 5' to 3' direction: a LAT promoter in operable combination with an *aprE*-5'UTR in operable combination with a LAT signal peptide in operable combination with an open reading frame encoding a mature G. *stearothermophilus* variant amylase, wherein the *aprE-5'UTR* has the sequence of SEQ ID NO: 9, optionally wherein the DNA molecule comprises sequence SEQ ID NO: 15.

In a fifth aspect, the invention provides a method for increasing expression of a protein of interest in a *Bacillus* host cell comprising: growing a modified *Bacillus* host cell in a suitable medium to express the protein of interest; wherein the modified *Bacillus* host cell is transformed with a vector comprising an expression cassette comprising in the 5' to 3' direction, at least (i) an aprE-5' UTR derived from a *Bacillus* spp. and (ii) a nucleic acid sequence encoding a protein of interest; wherein the expression of the protein of interest is increased by at least 5% relative to the expression of the same protein of interest expressed from a parental *Bacillus* host cell which does not comprises an aprE-5' UTR operably linked to the nucleic acid sequence encoding the protein of interest, and wherein the 5' UTR comprises the sequence of SEQ ID NO: 9.

In a sixth aspect, the invention provides a method for increasing expression of a protein of interest in a modified *Bacillus* host cell using no more than 50 ppm of chloramphenicol for amplification, the method comprising: growing a modified *Bacillus* host cell in a suitable medium to express the protein of interest; wherein the modified *Bacillus* host cell is transformed with a vector comprising an expression cassette comprising in the 5' to 3' direction, (i) an aprE-5' UTR derived from a *Bacillus* spp. and (ii) a nucleic acid sequence encoding for the protein of interest; wherein the expression of the protein of interest is at least 5% relative to the expression of the same protein of interest expressed from a parental *Bacillus* host cell which does not comprises an aprE-5' UTR operably linked to the nucleic acid sequence encoding the protein of interest, and wherein the 5' UTR comprises the sequence of SEQ ID NO: 9.

In some embodiments, the vector further comprises a promoter from a ribosomal RNA gene, optionally wherein the ribosomal RNA gene promoter is selected from the group consisting of P1 rrnB, P1 rrnI, P2 rrnI, P1 rrnE, P2 rrnE and P3 rrnE. In some embodiments, the vector further comprises a promoter from a ribosomal protein gene, optionally wherein the ribosomal protein gene promoter is selected from the group consisting of PrpsD, P1 rpsJ, P2 rpsJ and PrpoD. In some embodiments, the vector further comprises a promoter from a LAT (AmyL) gene. In some embodiments, the method further comprises recovering the protein of interest from the medium.

In some embodiments, the protein of interest is secreted from the bacterium. In some embodiments, the protein of interest accumulates within the bacterium. In some embodiments, the protein of interest is a wild-type *Bacillus* spp. enzyme or a variant *Bacillus* spp. enzyme.

In certain embodiments, the protein of interest is a homologous protein. In certain embodiments, the protein of interest is a heterologous protein. In certain embodiments, the protein of interest is an enzyme. In certain embodiments, the enzyme is selected from the group consisting of: acyl transferases, alpha-amylases, β-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-β-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, and xylosidases. In some embodiments, the enzyme is an amylase or a pullulanase.

In certain embodiments, the method further comprises recovering the protein of interest.

In certain embodiments, the method further comprises culturing the altered Gram positive bacterial cell under conditions such that the protein of interest is expressed by the altered Gram positive bacterial cell. In certain embodiments, the method further comprises recovering the protein of interest.

Aspects of the present invention include altered Gram positive bacterial cell produced by the methods described above.

### BRIEF DESCRIPTION OF THE DRAWINGS

Figure 1 is a plasmid pICatH-LATori1.
Figure 2 is a map of vector pHPLT-Blich-int-cassette.
Figure 3 is a map of vector pKB360-AprE-WT-AmyS variant.

### DETAILED DESCRIPTION OF THE INVENTION

Prior to describing the present compositions and methods in detail, the following terms are defined for clarity. Terms and abbreviations not defined should be accorded their ordinary meaning as used in the art. Unless defined otherwise herein, all technical and scientific terms used herein have the same meaning as commonly understood by one of ordinary skill in the art. Unless otherwise indicated, the practice of the present disclosure involves conventional techniques commonly used in molecular biology, protein engineering, and microbiology. Although any methods and materials similar or equivalent to those described herein find use in the practice of the present disclosure, some suitable methods and materials are described herein. The terms defined immediately below are more fully described by reference to the Specification as a whole.

As used herein, the singular "a," "an" and "the" includes the plural unless the context clearly indicates otherwise. Unless otherwise indicated, nucleic acid sequences are written left to right in 5' to 3' orientation; and amino acid sequences are written left to right in amino to carboxy orientation. It is to be understood that this disclosure is not limited to the particular methodology, protocols, and reagents described herein, absent an indication to the contrary.

It is intended that every maximum numerical limitation given throughout this Specification includes every lower numerical limitation, as if such lower numerical limitations were expressly written herein. Every minimum numerical limitation given throughout this Specification will include every higher numerical limitation, as if such higher numerical limitations were expressly written herein. Every numerical range given throughout this Specification will include every narrower numerical range that falls within such broader numerical range, as if such narrower numerical ranges were all expressly written herein.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

The citation of any publication is for its disclosure prior to the filing date and should not be construed as an admission that the present compositions and methods are not entitled to antedate such publication by virtue of prior invention. Further, the dates of publication provided may be different from the actual publication dates which may need to be independently confirmed.

### A. Definitions

As used herein, "host cell" refers to a cell that has the capacity to act as a host or expression vehicle for a newly introduced nucleic acid sequence.

In certain embodiments of the present invention, the host cells are bacterial cells, e.g., Gram-positive host cells *Bacillus sp.*

As used herein, "the genus *Bacillus*" or "*Bacillus sp*." includes all species within the genus "*Bacillus*," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B*. *thuringiensis.* It is recognized that the genus *Bacillus* continues to undergo taxonomical reorganization. Thus, it is intended that the genus include species that have been reclassified, including but not limited to such organisms as *B*. *stearothermophilus,* which is now named "*Geobacillus stearothermophilus*." The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

As used herein, "nucleic acid" refers to a nucleotide or polynucleotide sequence, and fragments or portions thereof, as well as to DNA, cDNA, and RNA of genomic or synthetic origin which may be double-stranded or single-stranded, whether representing the sense or antisense strand. It will be understood that as a result of the degeneracy of the genetic code, a multitude of nucleotide sequences may encode a given protein.

As used herein, the term "vector" refers to any nucleic acid that can be replicated in cells and can carry new genes or DNA segments into cells. Thus, the term refers to a nucleic acid construct designed for transfer between different host cells. An "expression vector" refers to a vector that has the ability to incorporate and express heterologous DNA fragments in a foreign cell. Many prokaryotic and eukaryotic expression vectors are commercially available. A "targeting vector" is a vector that includes polynucleotide sequences that are homologous to a region in the chromosome of a host cell into which it is transformed and that can drive homologous recombination at that region. Targeting vectors find use in introducing mutations into the chromosome of a cell through homologous recombination. In some embodiments, the targeting vector comprises other non-homologous sequences, e.g., added to the ends (i.e., stuffer sequences or flanking sequences). The ends can be closed such that the targeting vector forms a closed circle, such as, for example, insertion into a vector. Selection and/or construction of appropriate vectors are within the knowledge of those having skill in the art.

As used herein, the term "plasmid" refers to a circular double-stranded (ds) DNA construct used as a vector, and which forms an extrachromosomal self-replicating genetic element in many bacteria and eukaryotes. In some embodiments, plasmids may be incorporated into the genome of the host cell.

As defined herein, the term "LAT amylase" refers to the *B*. *licheniformis* α-amylase "AmyL", and as such, the terms may be used interchangeably.

By "purified" or "isolated" or "enriched" is meant that a biomolecule (e.g., a polypeptide or polynucleotide) is altered from its natural state by virtue of separating it from some or all of the naturally occurring constituents with which it is associated in nature. Such isolation or purification may be accomplished by art-recognized separation techniques such as ion exchange chromatography, affinity chromatography, hydrophobic separation, dialysis, protease treatment, ammonium sulfate precipitation or other protein salt precipitation, centrifugation, size exclusion chromatography, filtration, microfiltration, gel electrophoresis or separation on a gradient to remove whole cells, cell debris, impurities, extraneous proteins, or enzymes undesired in the final composition. It is further possible to then add constituents to a purified or isolated biomolecule composition which provide additional benefits, for example, activating agents, anti-inhibition agents, desirable ions, compounds to control pH or other enzymes or chemicals.

As used herein, the terms "enhanced", "improved" and "increased" when referring to expression of a biomolecule of interest (e.g., a protein on interest) are used interchangeably herein to indicate that expression of the biomolecule is above the level of expression in a corresponding host strain (e.g., a wild type and/or a parental strain) that has not been altered according to the teachings herein but has been grown under essentially the same growth conditions.

As used herein the term "expression" when applied to a protein refers to a process by which a protein is produced based on the nucleic acid sequence of a gene and thus includes both transcription and translation.

As used herein in the context of introducing a polynucleotide into a cell, the term "introduced" refers to any method suitable for transferring the polynucleotide into the cell. Such methods for introduction include but are not limited to protoplast fusion, transfection, transformation, conjugation, and transduction (*See e.g.,* Ferrari et al., "Genetics," in Hardwood et al, (eds.), Bacillus, Plenum Publishing Corp., pages 57-72, [1989]).

As used herein, the terms "transformed" and "stably transformed" refers to a cell into which a polynucleotide sequence has been introduced by human intervention. The polynucleotide can be integrated into the genome of the cell or be present as an episomal plasmid that is maintained for at least two generations.

As used herein, the terms "selectable marker" or "selective marker" refer to a nucleic acid (*e.g*., a gene) capable of expression in host cell which allows for ease of selection of those hosts containing the nucleic acid. Examples of such selectable markers include but are not limited to antimicrobials. Thus, the term "selectable marker" refers to genes that provide an indication that a host cell has taken up an incoming DNA of interest or some other reaction has occurred. Typically, selectable markers are genes that confer antimicrobial resistance or a metabolic advantage on the host cell to allow cells containing the exogenous DNA to be distinguished from cells that have not received any exogenous sequence during the transformation. Other markers useful in accordance with the invention include, but are not limited to auxotrophic markers, such as tryptophan; and detection markers, such as β- galactosidase.

As used herein, the term "promoter" refers to a nucleic acid sequence that functions to direct transcription of a downstream gene. In embodiments, the promoter is appropriate to the host cell in which the target gene is being expressed. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

As used herein, "functionally attached" or "operably linked" means that a regulatory region or functional domain having a known or desired activity, such as a promoter, terminator, signal sequence or enhancer region, is attached to or linked to a target (e.g., a gene or polypeptide) in such a manner as to allow the regulatory region or functional domain to control the expression, secretion or function of that target according to its known or desired activity.

The term "genetic alteration" or "genetic change" when used to describe a recombinant cell means that the cell has at least one genetic difference as compared to a parent cell. The one or more genetic difference may be a chromosomal mutation (e.g., an insertion, a deletion, substitution, inversion, replacement of a chromosomal region with another (e.g., replacement of a chromosomal prompter with a heterologous promoter), etc.) and/or the introduction of an extra-chromosomal polynucleotide (e.g., a plasmid). In some embodiments, an extra-chromosomal polynucleotide may be integrated into the chromosome of the host cell to generate a stable transfectant/transformant.

"Inactivation" of a gene means that the expression of a gene or the activity of its encoded biomolecule is blocked or is otherwise unable to exert its known function. Inactivation can occur via any suitable means, e.g., via a genetic alteration as described above. In one embodiment, the expression product of an inactivated gene is a truncated protein with a corresponding change in the biological activity of the protein.

In some embodiments, inactivation is achieved by deletion. In some embodiments, the region targeted for deletion (e.g., a gene) is deleted by homologous recombination. For example, a DNA construct comprising an incoming sequence having a selective marker flanked on each side by sequences that are homologous to the region targeted for deletion is used (where the sequences may be referred to herein as a "homology box"). The DNA construct aligns with the homologous sequences of the host chromosome and in a double crossover event the region targeted for deletion is excised out of the host chromosome.

An "insertion" or "addition" is a change in a nucleotide or amino acid sequence which has resulted in the addition of one or more nucleotides or amino acid residues, respectively, as compared to the naturally occurring or parental sequence.

As used herein, a "substitution" results from the replacement of one or more nucleotides or amino acids by different nucleotides or amino acids, respectively.

Methods of mutating genes are well known in the art and include but are not limited to site-directed mutation, generation of random mutations, and gapped-duplex approaches (See *e.g.,* U.S. Pat. 4,760,025; Moring et al., Biotech. 2:646 [1984]; and Kramer et al., Nucleic Acids Res., 12:9441 [1984]).

As used herein, "homologous genes" refers to a pair or more of genes from different, but usually related species, which correspond to each other and which are identical or very similar to each other. The term encompasses genes that are separated by speciation (*i.e.*, the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene (*i.e.*, a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "homology" refers to sequence similarity or identity, with identity being preferred. This homology is determined using standard techniques known in the art (See *e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, Wl); and Devereux et al., Nucl. Acid Res., 12:387-395 [1984]).

As used herein, an "analogous sequence" is one wherein the function of the gene is essentially the same as the gene designated from *Bacillus subtilis* strain 168. Additionally, analogous genes include at least 60%, 65%, 70%, 75%, 80%, 85%, 90%, 95%, 97%, 98%, 99% or 100% sequence identity with the sequence of the *Bacillus subtilis* strain 168 gene. Alternately, analogous sequences have an alignment of between 70 to 100% of the genes found in the *B. subtilis* 168 region and/or have at least between 5-10 genes found in the region aligned with the genes in the *B. subtilis* 168 chromosome. In additional embodiments more than one of the above properties applies to the sequence. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps.

Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). A particularly useful BLAST program is the WU-BLAST-2 program (See, Altschul et al., Meth. Enzymol. 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

As used herein, "percent (%) sequence identity" with respect to the amino acid or nucleotide sequences identified herein is defined as the percentage of amino acid residues or nucleotides in a candidate sequence that are identical with the amino acid residues or nucleotides in a sequence of interest, after aligning the sequences and introducing gaps, if necessary, to achieve the maximum percent sequence identity, and not considering any conservative substitutions as part of the sequence identity.

By "homologue" (or "homolog") shall mean an entity having a specified degree of identity with the subject amino acid sequences and the subject nucleotide sequences. A homologous sequence can include an amino acid sequence that is at least 60%, 65%, 70%, 75%, 80%, 85%, 86%, 87%, 88%, 89%, 90%, 91%, 92%, 93%, 94%, 95%, 96%, 97%, 98% or even 99% identical to the subject sequence, using conventional sequence alignment tools (*e.g.*, Clustal, BLAST, and the like). Typically, homologues will include the same active site residues as the subject amino acid sequence, unless otherwise specified.

Methods for performing sequence alignment and determining sequence identity are known to the skilled artisan, may be performed without undue experimentation, and calculations of identity values may be obtained with definiteness. See, for example, Ausubel et al., eds. (1995) Current Protocols in Molecular Biology, Chapter 19 (Greene Publishing and Wiley-Interscience, New York); and the ALIGN program (Dayhoff (1978) in Atlas of Protein Sequence and Structure 5:Suppl. 3 (National Biomedical Research Foundation, Washington, D.C.). A number of algorithms are available for aligning sequences and determining sequence identity and include, for example, the homology alignment algorithm of Needleman et al. (1970) J. Mol. Biol. 48:443; the local homology algorithm of Smith et al. (1981) Adv. Appl. Math. 2:482; the search for similarity method of Pearson et al. (1988) Proc. Natl. Acad. Sci. 85:2444; the Smith-Waterman algorithm (Meth. Mol. Biol. 70:173-187 (1997); and BLASTP, BLASTN, and BLASTX algorithms (see Altschul et al. (1990) J. Mol. Biol. 215:403-410).

Computerized programs using these algorithms are also available, and include, but are not limited to: ALIGN or Megalign (DNASTAR) software, or WU-BLAST-2 (Altschul et al., Meth. Enzymol., 266:460-480 (1996)); or GAP, BESTFIT, BLAST, FASTA, and TFASTA, available in the Genetics Computing Group (GCG) package, Version 8, Madison, Wisconsin, USA; and CLUSTAL in the PC/Gene program by Intelligenetics, Mountain View, California. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can determined by using Clustal W (Thompson J.D. et al. (1994) Nucleic Acids Res. 22:4673-4680) with default parameters.

As used herein, the term "hybridization" refers to the process by which a strand of nucleic acid joins with a complementary strand through base pairing, as known in the art.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while intermediate or low stringency hybridization can be used to identify or detect polynucleotide sequence homologs.

Moderate and high stringency hybridization conditions are well known in the art. An example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. as necessary to accommodate factors such as probe length and the like.

The term "recombinant," when used in reference to a biological component or composition (e.g., a cell, nucleic acid, polypeptide/enzyme, vector, etc.) indicates that the biological component or composition is in a state that is not found in nature. In other words, the biological component or composition has been modified by human intervention from its natural state. For example, a recombinant cell encompass a cell that expresses one or more genes that are not found in its native parent (i.e., non-recombinant) cell, a cell that expresses one or more native genes in an amount that is different than its native parent cell, and/or a cell that expresses one or more native genes under different conditions than its native parent cell. Recombinant nucleic acids may differ from a native sequence by one or more nucleotides, be operably linked to heterologous sequences (e.g., a heterologous promoter, a sequence encoding a non-native or variant signal sequence, etc.), be devoid of intronic sequences, and/or be in an isolated form. Recombinant polypeptides/enzymes may differ from a native sequence by one or more amino acids, may be fused with heterologous sequences, may be truncated or have internal deletions of amino acids, may be expressed in a manner not found in a native cell (e.g., from a recombinant cell that over-expresses the polypeptide due to the presence in the cell of an expression vector encoding the polypeptide), and/or be in an isolated form. It is emphasized that in some embodiments, a recombinant polynucleotide or polypeptide/enzyme has a sequence that is identical to its wild-type counterpart but is in a non-native form (e.g., in an isolated or enriched form).

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, a "flanking sequence" refers to any sequence that is either upstream or downstream of the sequence being discussed (*e.g.*, for genes A-B-C, gene B is flanked by the A and C gene sequences). In an embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in embodiments, it is on each side of the sequence being flanked. The sequence of each homology box is homologous to a sequence in the *Bacillus* chromosome. These sequences direct where in the *Bacillus* chromosome the new construct gets integrated and what part of the *Bacillus* chromosome will be replaced by the incoming sequence. In an embodiment, the 5' and 3' ends of a selective marker are flanked by a polynucleotide sequence comprising a section of the inactivating chromosomal segment. In some embodiments, a flanking sequence is present on only a single side (either 3' or 5'), while in embodiments, it is present on each side of the sequence being flanked.

As used herein, the terms "amplifiable marker," "amplifiable gene," and "amplification vector" refer to a gene or a vector encoding a gene which permits the amplification of that gene under appropriate growth conditions.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (See *e.g.,* Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (*See*, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See*, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e*., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e*., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of, among others, U.S. Patent Nos. 4,683,195 4,683,202, and 4,965,188 which include methods for increasing the concentration of a segment of a target sequence in a mixture of genomic DNA without cloning or purification.

As used herein, "genetically altered host strain" (e.g., a genetically altered Bacillus strain) refers to a genetically engineered host cell, also called a recombinant host cell. In some embodiments, the genetically altered host cell has enhanced (increased) expression of a protein of interest as compared to the expression and/or production of the same protein of interest in a corresponding unaltered host strain grown under essentially the same growth conditions. In some embodiments, the altered strains are genetically engineered *Bacillus* sp. having one or more deleted indigenous chromosomal regions or fragments thereof, where a protein of interest has an enhanced level of expression or production, as compared to a corresponding unaltered *Bacillus* host strain grown under essentially the same growth conditions.

As used herein, a "corresponding unaltered *Bacillus* strain" and the like is the host strain (*e.g.*, the originating (parental) and/or wild-type strain) which does not have the indicated genetic alteration.

As used herein, the term "chromosomal integration" refers to the process whereby the incoming sequence is introduced into the chromosome of a host cell (*e.g., Bacillus*). The homologous regions of the transforming DNA align with homologous regions of the chromosome. Subsequently, the sequence between the homology boxes is replaced by the incoming sequence in a double crossover (*i.e.*, homologous recombination). In some embodiments of the present invention, homologous sections of an inactivating chromosomal segment of a DNA construct align with the flanking homologous regions of the indigenous chromosomal region of the *Bacillus* chromosome. Subsequently, the indigenous chromosomal region is deleted by the DNA construct in a double crossover (*i.e.*, homologous recombination).

"Homologous recombination" means the exchange of DNA fragments between two DNA molecules or paired chromosomes at the site of identical or nearly identical nucleotide sequences. In an embodiment, chromosomal integration is homologous recombination.

"Homologous sequences" as used herein means a nucleic acid or polypeptide sequence having 100%, 99%, 98%, 97%, 96%, 95%, 94%, 93%, 92%, 91%, 90%, 88%, 85%, 80%, 75%, or 70% sequence identity to another nucleic acid or polypeptide sequence when optimally aligned for comparison. In some embodiments, homologous sequences have between 85% and 100% sequence identity, while in other embodiments there is between 90% and 100% sequence identity, and in more embodiments, there is 95% and 100% sequence identity.

As used herein "amino acid" refers to peptide or protein sequences or portions thereof. The terms "protein", "peptide" and "polypeptide" are used interchangeably.

As used herein, "protein of interest" and "polypeptide of interest" refer to a protein/polypeptide that is desired and/or being assessed. In some embodiments, the protein of interest is intracellular, while in other embodiments, it is a secreted polypeptide. Particularly polypeptides include enzymes, including, but not limited to those selected from amylolytic enzymes, proteolytic enzymes, cellulolytic enzymes, oxidoreductase enzymes and plant cell-wall degrading enzymes. More particularly, these enzymes include, but are not limited to acyl transferases, alpha-amylases, β-amylases, alpha-galactosidases, arabinosidases, aryl esterases, beta-galactosidases, carrageenases, catalases, cellobiohydrolases, cellulases, chondroitinases, cutinases, endo-beta-1, 4-glucanases, endo-p-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, and xylosidases. In some embodiments, the protein of interest is a secreted polypeptide which is fused to a signal peptide (*i.e.*, an amino-terminal extension on a protein to be secreted). Nearly all secreted proteins use an amino- terminal protein extension which plays a crucial role in the targeting to and translocation of precursor proteins across the membrane. This extension may be proteolytically removed by a signal peptidase during or following membrane transfer.

In some embodiments of the present invention, the polypeptide or protein of interest is selected from hormones, antibodies, growth factors, receptors, etc. Hormones encompassed by the present invention include but are not limited to, follicle-stimulating hormone, luteinizing hormone, corticotropin-releasing factor, somatostatin, gonadotropin hormone, vasopressin, oxytocin, erythropoietin, insulin and the like. Growth factors include, but are not limited to platelet-derived growth factor, insulin-like growth factors, epidermal growth factor, nerve growth factor, fibroblast growth factor, transforming growth factors, cytokines, such as interleukins (*e.g.*, IL-1 through IL-13), interferons, colony stimulating factors, and the like. Antibodies include but are not limited to immunoglobulins obtained directly from any species from which it is desirable to produce antibodies. In addition, the present invention encompasses modified antibodies. Polyclonal and monoclonal antibodies are also encompassed by the present invention. In particularly embodiments, the antibodies are human antibodies.

As used herein, a "derivative" or "variant" of a polypeptide means a polypeptide, which is derived from a precursor polypeptide (e.g., the native polypeptide) by addition of one or more amino acids to either or both the C- and N-terminal ends, substitution of one or more amino acids at one or a number of different sites in the amino acid sequence, deletion of one or more amino acids at either or both ends of the polypeptide or at one or more sites in the amino acid sequence, insertion of one or more amino acids at one or more sites in the amino acid sequence, and any combination thereof. The preparation of a derivative or variant of a polypeptide may be achieved in any convenient manner, e.g., by modifying a DNA sequence which encodes the native polypeptides, transformation of that DNA sequence into a suitable host, and expression of the modified DNA sequence to form the derivative/variant polypeptide. Derivatives or variants further include polypeptides that are chemically modified.

As used herein, the term "heterologous protein" refers to a protein or polypeptide that does not naturally occur in the host cell. In some embodiments, the genes encoding the proteins are naturally occurring genes, while in other embodiments; mutated and/or synthetic genes may be used. In some embodiments, the DNA encoding the heterologous protein is linked with a 5' UTR that is heterologous in nature, meaning that the 5' UTR and the DNA coding the protein of interest are from different genes or different organisms. Thus, the term heterologous is used in its broadest sense that two elements are not from the same origin (e.g., species, cell, nucleic acid, etc.).

As used herein, "homologous protein" refers to a protein or polypeptide native or naturally occurring in a cell. In embodiments, the cell is a Gram-positive cell, while in particularly embodiments; the cell is a *Bacillus* host cell. In alternative embodiments, the homologous protein is a native protein produced by other organisms, including but not limited to *E. coli.* The invention encompasses host cells producing the homologous protein via recombinant DNA technology.

### B. UnTranslated Regions (UTRs)

UnTranslated Region or "UTR" is a sequence of am mRNA or the corresponding DNA that is generally not translated. UTRs may be at the 5' or 3' end of an mRNA. The 5' untranslated region (5' UTR) (also known as a *Leader Sequence* or *Leader RNA*) is the region of an mRNA that is upstream from the initiation codon. This region is important for the regulation of translation of a transcript by differing mechanisms in viruses, prokaryotes and eukaryotes. While called untranslated, the 5' UTR or a portion of it is sometimes translated into a protein product. This product may then regulate the translation of the main coding sequence of the mRNA. In many other organisms, however, the 5' UTR is untranslated, instead forming complex secondary structure to regulate translation. The corresponding DNA sequence is also generally referred to as 5' or 3' UTR sequences.

5' UTRs may begin at the transcription start site and may end at or near translation start site or the first codon of the coding region. The 5'UTRs vary in length from 3-10 nucleotides to over several thousands of nucleotides. The 5' UTRs generally contain ribosomal binding sites, cis-acting regulatory elements, as well as other regulatory elements.

Various genes from Bacillus bacteria contain 5' UTRs. The LAT (alpha amylase) gene from *B*. *licheniformis* comprises the following 5' UTR:
GTTTCACATTGAAAGGGGAGGAGAATC (SEQ ID NO: 8), while the *aprE* gene from *B*. *subtilis* comprises the following 5' UTR:
GACAGAATAGTCTTTTAAGTAAGTCTACTCTGAATTTTTTTAAAAGGAGAGGGTAAAGA (SEQ ID NO: 2).

Hambraeus *et al.* have shown that the 58 nucleotide long leader sequence (5' UTR) of the aprE gene from B. subtilis is determinant of mRNA stability (Hambraeus et al. Microbiology (2002) 148: 1795-1803). They showed that this region forms a stem-loop structure at the 5' end and together with an intact ribosomal binding site (RBS) lead to the enhanced stability of the mRNA. However, Hambraeus et al. did not study nor teach, nor suggest; and furthermore, do not realize and therefore, do not motivate one skilled in the art that use of a strong 5' UTR in an expression vector may result in higher expression of a protein of interest. The present inventors have now found that a strong 5' UTR in an expression vector does result in higher expression of proteins of interest in a modified host cell.

### C. Promoters

Specifically, examples of suitable promoters for use in bacterial host cells include, but are not limited to, for example, the *PamyE,* P*amy*Q, *PamyL*, P*pstS,* Psac*B*, P*SPAC*, P*AprE,* P*Veg*, P*Hpall* promoters, the promoter of the *B*. *stearothermophilus* maltogenic amylase gene, the *B. amyloliquefaciens* (BAN) amylase gene, the *B*. *subtilis* alkaline protease gene, the *B*. *clausii* alkaline protease gene the *B*. *pumilis* xylosidase gene, the *B*. *thuringiensis* crylllA, and the *B*. *licheniformis* alpha-amylase gene. Additional promoters include, but are not limited to the A4 promoter, as well as phage Lambda PR or PL promoters, and the E. coli *lac, trp* or *tac* promoters. Additionally, ribosomal protein and RNA promoters have been found to be useful in heterologous protein production (See, for example, WO 2013/086219, ). The following promoters are contemplated.

| **Table 1-1: List of promoters (the -35 and -10 consensus sequences are bold and underlined)** | | |
|---|---|---|
| Ribosomal RNA Promoters | | |
| **Name** | **Sequence** | **SEQ ID NO** |
| P1 *rmB* | ATAGATTTTTTTTAAAAAACTATTGCAATAAATAAATACAG GTGTTATATTATTAAACG TCGCTG | 18 |
| P1 *rmI* | CACATACAGCCTAAATTGGGTGTTGACCTTTTGATAATAT CCGTGATATATTATTATTCG TCGCTG | 19 |
| P2 *rmI* | TTAAATACTTTGAAAAAAGTTGTTGACTTAAAAGAAGCTAA ATGTTATAGTAATAAAG CTGCTT | 20 |
| P1 *rmE* | ATAAAAAAATACAGGAAAAGTGTTGACCAAATAAAACAGG CATGGTATATTATTAAACG TCGCTG | 21 |
| P2 *rmE* | AACAAAAAAGTTTTCCTAAGGTGTTTACAAGATTTTAAAAA TGTGTATAATAAGAAAAG TCGAAT | 22 |
| P3 *rmE* | TCGAAAAAACATTAAAAAACTTCTTGACTCAACATCAAATG ATAGTATGATAGTTAAG TCGCTC | 23 |

**Table 2-1: Promoter sequences are shown for rpsD, rpsJ, and rpoD (P1). -35 and -10 sequences are shown in bold and underlined for each promoter. For rpsJ, two promoters are available (P1 and P2). The -35 and -10 sequences for rpsJ P1 are upstream (i.e., 5') of the -35 and -10 sequences for rpsJ P2 sequences.**

| **Table 2-1: List of promoters (the -35 and -10 consensus sequences are bold and underlined)** | | |
|---|---|---|
| **Name** | **Sequence** | **SEQ ID NO** |
| Ribosomal protein promoters | | |
| *rpsD* | GTTTTTATCACCTAAAAGTTTACCACTAATTTTTGTTTATTAT ATCATAAACGGTGAAGCAATAATGGAGGAATGGTTGACTTC AAAACAAATAAATTATATAATGACCTTT | 24 |
| *rpsJ* | GTACCGTGTGTTTTCATTTCAGGGAAACATGACTTAATTGTT CCTGCAGAAATATCGAAACAGTATTATCAAGAACTTGAGGC ACCTGAAAAGCGCTGGTTTCAATTTGAGAATTCAGCTCACA CCCCGCATATTGAGGAGCCATCATTATTCGCGAACACATTA AGTCGGCATGCACGCAACCATTTATGATAGATCCTTGATAA ATAAGAAAAACCCCTGTATAATAAAAAAAGTGTGCAAATGA TGCATATTTTAAATAAGTCTTGCAACATGCGCCTATTTTCTG TATAATGGTGTATA | 25 |

| Sigma factor promoter | | |
|---|---|---|
| *rpoD* (P1) | AACATATAACTCAGGACGCTCTATCCTGGGTTTTTGGCTGT GCCAAAAGGGAATAATGAAAAACAATAGCATCTTTGTGAA GTTTGTATTATAATAAAAAATT | 26 |

The unexpectedly high level of expression of a nucleic acid sequence coding for a heterologous protein of interest when using ribosomal promoters has several benefits. In one embodiment, expressing a coding sequence of interest with a ribosomal promoter allows for increased level of expression of a coding sequence of interest when compared to expression of the coding sequence of interest from its native promoter. An increased level of expression is particularly useful for transcripts that are unstable.

In another embodiment, expressing a coding sequence of interest with a ribosomal promoter allows for increased level of expression of a coding sequence of interest without amplification of an expression construct comprising the ribosomal promoter. When using other expression constructs in the art, in order to achieve high expression levels of a coding sequence of interest, amplification of the expression construct is often required. The expression levels achieved with the ribosomal promoters described herein, however, are high enough that amplification of the expression construct may not be necessary (but may be used to achieve even higher expressions of protein of interest). This provides several benefits. First, host strains are more stable because they do not undergo the loss of the amplified expression construct. Also, if an expression construct does not need to be amplified, strain construction is more efficient. Thus, time, money and materials are saved.

### D. Coding Sequences of Interest

The promoters encompassed by the invention are operably linked to a nucleic acid encoding a protein of interest (i.e., a coding sequence of interest). The polypeptide encoded by the coding sequence may be an enzyme, a hormone, a growth factor, a cytokine, an antibiotic or portion thereof, a receptor or portion thereof, a reporter gene (e.g., green fluorescent protein) or other secondary metabolites.

In some embodiments, the enzyme is a protease, cellulase, hemicellulase, xylanase, amylase, glucoamylase, cutinase, phytase, laccase, lipase, isomerase, esterase, mannanase, carbohydrase, hydrolase, oxidase, permease, pullulanase, reductase, epimerase, tautomerase, transferase, kinase, phosphatase, or the like originating from bacteria or fungi.

In other embodiments, the enzyme is a protease, such as a serine, metallo, thiol or acid protease. In some embodiments, the protease will be a serine protease (e.g., subtilisin). Serine proteases are described in Markland, et al. (1983) Honne-Seyler's Z Physiol. Chem 364:1537-1540; Drenth, J. et al. (1972) Eur. J. Biochem. 26:177-181; U.S. Patent Nos. 4,760,025 (RE 34,606), 5,182,204 and 6,312,936 and EP 0 323,299. Examples of alkaline proteases include subtilisins, especially those derived from Bacillus (*e.g.*, subtilisin, lentus, amyloliquefaciens, subtilisin Carlsberg, subtilisin 309, subtilisin 147 and subtilisin 168). Proteases that may be used in the invention are also described in, for example, U.S. Patent Publication No. 2010/0152088 and International Publication No. WO 2010/056635. Means for measuring proteolytic activity are disclosed in K. M. Kalisz, "Microbial Proteinases" ADVANCES IN BIOCHEMICAL ENGINEERING AND BIOTECHNOLOGY, A. Fiecht Ed. 1988.

In other embodiments, the enzyme is an amylase, such as an amylase derived from *Bacillus* (such as *B. licheniformis* LAT amylase), an amylase derived from *Geobacillus* (such as *G. stearothermophilus*) or *Trichoderma* (such as *T. reesei*). Bacterial and fungal amylases are described in, for example, U.S. Patent No. 8,058,033, U.S. Patent Publication No. 2010/0015686, U.S. Patent Publication No. 2009/0314286, UK application No. 1011513.7, and International Application No. PCT/IB2011/053018.

In other embodiments, the enzyme is a xylanase. In certain embodiments, the xylanase is derived from *Trichoderma* (such as *T. reesei*)*.* Bacterial and fungal xylanases are described in, for example, International Publication No. WO 2001/027252 and U.S. Patent No. 7,718,411.

In other embodiments, the enzyme is a phytase. In certain embodiments, the phytase is derived from *Citrobacter* (such as *C.freundii*) or *E*. *coli.* In other embodiments, they phytase may be a *Buttiauxella* phytase such as a *Buttiauxella agrestis* phytase. Phytases are described in, for example, International Publication Nos. WO 2006/043178, WO 2006/038062, WO 2008/097619, WO 2009/129489, WO 2006/038128, WO 2008/092901, WO 2009/129489, and WO 2010/122532.

In some embodiments, the enzyme is a cellulase. Cellulases are enzymes that hydrolyze the beta-D-glucosidic linkages in celluloses. Cellulolytic enzymes have been traditionally divided into three major classes: endoglucanases, exoglucanases or cellobiohydrolases and beta-glucosidases (Knowles, J. et al., TIBTECH 5:255-261 (1987)). Numerous cellulases have been described in the scientific literature, and are well known to one skilled in the art.

In some embodiments, the hormone is a follicle-stimulating hormone, luteinizing hormone, corticotropin-releasing factor, somatostatin, gonadotropin hormone, vasopressin, oxytocin, erythropoietin, insulin and the like.

In some embodiments, the growth factor, which is a protein that binds to receptors on the cell surface with the primary result of activating cellular proliferation and/or differentiation, include platelet-derived growth factor, epidermal growth factor, nerve growth factor, fibroblast growth factor, insulin-like growth factors, transforming growth factors and the like.

In some embodiments, the growth factor is a cytokine. Cytokines include but are not limited to colony stimulating factors, the interleukins (IL-I (alpha and beta), IL-2 through IL-13) and the interferons (alpha, beta and gamma).

In some embodiments, the antibodies include, but are not limited to, immunoglobulins from any species from which it is desirable to produce large quantities, It is especially preferred that the antibodies are human antibodies. Immunoglobulins may be from any class, i.e. G, A, M, E or D.

The coding sequence may be either native or heterologous to a host cell. In addition, the coding sequence may encode a full-length protein, or a truncated form of a full-length protein. The invention is not limited to a particular coding sequence but encompasses numerous coding sequences, which are operably linked to a promoter of the invention.

### E. Signal Sequences

In some embodiments, especially when the coding sequence of interest codes for an extracellular enzyme, such as a cellulase, protease or starch degrading enzyme, a signal sequence may be linked to the N-terminal portion of the coding sequence. The signal may be used to facilitate the secretion of a DNA sequence. The signal sequence may be endogenous or exogenous to the host organism. The signal sequence may be one normally associated with the encoded polypeptide. In some embodiments, the signal sequence may be altered or modified as described in International Patent Publication Nos. WO2011/014278 and WO2010/123754. In some embodiments, the signal sequence comprises a signal sequence from a *Streptomyces* cellulase gene. In one embodiment, a preferred signal sequence is a S. *lividans* cellulase, celA (Bently et al., (2002) Nature 417:141-147). However, one skilled in the art is aware of numerous signal peptides (*e.g.*, a *B. licheniformis* amylase signal peptide) which may be used depending on a protein to be expressed and secreted in a host organism.

### F. DNA Constructs and Vectors

The nucleic acid construct of the invention comprising a coding region of interest may be prepared synthetically by established standard methods, e.g., the phosphoramidite method described by Beaucage and Caruthers, (1981) Tetrahedron Letters 22:1859-1869, or the method described by Matthes et al., (1984) EMBO Journal 3: 801-805. The nucleic acid construct may be of mixed synthetic and genomic origin and may be prepared by ligating fragments of synthetic or genomic DNA. The nucleic acid construct may also be prepared by polymerase chain reaction using specific primers, for instance as described in U.S. Patent No. 4,683,202 or Saiki et al., Science 239 (1988), 487-491.

A DNA construct of the invention may be inserted into a vector, such as an expression vector. A variety of vectors suitable for the cloning, transformation and expression of polypeptides in fungus, yeast and bacteria are known by those of skill in the art. Typically, the vector or cassette will comprise a promoter of the invention, optionally a signal sequence, a coding region of interest and a terminator sequence. In preferred embodiments, the vector will include one or more cloning sites located between the signal sequence and the terminator sequences.

### G. Transformation

A vector of the invention will be transformed into a host cell. General transformation techniques are known in the art (Ausubel et al., 1994, CURRENT PROTOCOLS IN MOLECULAR BIOLOGY and Campbell et al., 1989 Curr. Genet 16:53-56). Some of these general techniques include, but are not limited to the use of a particle or gene gun (biolistics), permeabilization of filamentous fungi cells walls prior to the transformation process (e.g., by use of high concentrations of alkali, e.g., 0.05 M to 0.4 M CaCl₂ or lithium acetate), protoplast fusion, electroporation, or agrobacterium mediated transformation (U.S. Pat. No. 6,255,115) and the treatment of protoplasts or spheroplasts with polyethylene glycol and CaCl.sub.2 is described in Campbell, et al., (1989) Curr. Genet. 16:53-56, 1989 and Penttila, M. et al., (1988) Gene, 63:11-22.

Transformation and expression methods for bacteria are disclosed in Brigidi, DeRossi, Bertarini, Riccardi and Matteuzzi, (1990), FEMS Microbiol. Lett. 55: 135-138. A preferred general transformation and expression protocol for protease deleted Bacillus strains is provided in Ferrari et al., U.S. Patent No. 5,264,366.

Transformation and expression in Streptomyces can be found in Hopwood et al., GENETIC MANIPULATION OF STREPTOMYCES: A LABORATORY MANUAL, (1985) John Innis Foundation, Norwich UK.

In other embodiments, transformation and expression in *Aspergillus* and *Trichoderma* is described in, for example U.S. Patent No. 5,364,770; U.S. Patent No. 6,022,725; and Nevalainen et al., 1992, The Molecular Biology of Trichoderma and its Application to the Expression of Both Homologous and Heterologous Genes, in MOLECULAR INDUSTRIAL MYCOLOGY, Eds. Leon and Berka, Marcel Dekker, Inc. pp. 129-148.

### H. Host Cells

Host cells that may be used according to the invention include both bacterial and fungal cells. Preferred fungal host cells include filamentous fungal cells such as *Aspergillus* and *Trichoderma* cells. Preferred bacterial host cells include both Gram positive and Gram negative cells, including *Bacillus*, *Mycobacterium*, *Actinomyces* and *Streptomyces* cells. Host cells also include, without limitation, *E*. *coli*, *Pseudomonas spp.* (e.g., *P*. *aeruginosa* and *P*. *alcaligenes*), *Streptomyces spp.,* (e.g., *Streptomyces lividans*), *B. subtilis*, *B. licheniformis, B. lentus, B. brevis, B. stearothermophilus, B. alkalophilus, B. amyloliquefaciens*, *B. coagulans*, *B. circulans*, *B. lautus*, *B. megatherium*, and *B*. *thuringiensis.*

### I. Cell Culture

Host cells and transformed cells can be cultured in conventional nutrient media. The culture media for transformed host cells may be modified as appropriate for activating promoters and selecting transformants. The specific culture conditions, such as temperature, pH and the like, may be those that are used for the host cell selected for expression, and will be apparent to those skilled in the art. In addition, preferred culture conditions may be found in the scientific literature such as Sambrook, (1982) supra; Kieser, T, M J. Bibb, M J. Buttner, K F Chater, and D. A. Hopwood (2000) PRACTICAL STREPTOMYCES GENETICS. John Innes Foundation, Norwich UK; Harwood, et al., (1990) MOLECULAR BIOLOGICAL METHODS FOR BACILLUS, John Wiley and/or from the American Type Culture Collection (ATCC; "http://www.atcc.org/"). Stable transformants of fungal host cells, such as Trichoderma cells can generally be distinguished from unstable transformants by their faster growth rate or the formation of circular colonies with a smooth rather than ragged outline on solid culture medium.

### J. Recovery of Expressed Polypeptides

A polypeptide produced by the transformed host cell may be recovered from the culture medium by conventional procedures including separating the host cells from the medium by centrifugation or filtration, or if necessary, disrupting the cells and removing the supernatant from the cellular fraction and debris. Typically after clarification, the proteinaceous components of the supernatant or filtrate are precipitated by means of a salt, e.g., ammonium sulfate. The precipitated proteins are then solubilized and may be purified by a variety of chromatographic procedures, e.g., ion exchange chromatography, gel filtration chromatography, affinity chromatography, and other art-recognized procedures.

### K. Construct Assembly

In one general embodiment, the present invention involves assembling a DNA construct in vitro, followed by direct cloning of such construct into competent host cells (e.g., Bacillus host cells) such that the construct becomes integrated into the host genome. For example, in some embodiments PCR fusion and/or ligation are employed to assemble a DNA construct in vitro. In a preferred embodiment, the DNA construct is a non-plasmid DNA construct. In another embodiment, the DNA construct comprises a DNA into which a mutation has been introduced. This construct is then used to transform host cells. In this regard, highly competent mutants of a host cell (e.g., Bacillus) are preferably employed to facilitate the direct cloning of the constructs into the cells. For example, Bacillus carrying the *comK* gene under the control of a xylose-inducible promoter (*Pxyl-comK*) can be reliably transformed with very high efficiency, as described herein. Any suitable method known in the art may be used to transform the cells. The DNA construct may be inserted into a vector (i.e., a plasmid), prior to transformation. In some preferred embodiments, the circular plasmid is cut using an appropriate restriction enzyme (i.e., one that does not disrupt the DNA construct). Thus, in some embodiments, circular plasmids find use with the present invention. However, in alternative embodiments, linear plasmids are used. In some embodiments, the DNA construct (i.e., the PCR product) is used without the presence of plasmid DNA.

In order to further illustrate the present invention and advantages thereof, the following specific examples are given with the understanding that they are being offered to illustrate the present invention and should not be construed in any way as limiting its scope.

Whether the DNA construct is incorporated into a vector or used without the presence of plasmid DNA, it is used to transform microorganisms. It is contemplated that any suitable method for transformation will find use with the present invention. In embodiments, at least one copy of the DNA construct is integrated into the host *Bacillus* chromosome. In some embodiments, one or more DNA constructs of the invention are used to transform host cells.

### EXPERIMENTAL

The following Examples are provided in order to demonstrate and further illustrate certain embodiments and aspects of the present invention and are not to be construed as limiting the scope thereof.

In the experimental disclosure which follows, certain of the following abbreviations apply: °C (degrees Centigrade); rpm (revolutions per minute); µg (micrograms); mg (milligrams); µl (microliters); ml (milliliters); mM (millimolar); µM (micromolar); sec (seconds); min(s) (minute/minutes); hr(s) (hour/hours); OD₂₈₀ (optical density at 280 nm); OD₆₀₀ (optical density at 600 nm); PCR (polymerase chain reaction); RT-PCR (reverse transcription PCR); SDS (sodium dodecyl sulfate); Abs (absorbance).

### Comparative Example 1

### Improved production of B. licheniformis alpha-amylase (LAT) by the aprE 5'UTR

An expression construct containing the *Bacillus licheniformis* alpha-amylase (LAT) gene with the native LAT promoter, LAT 5'UTR, LAT signal peptide sequence and LAT transcription terminator was cloned into the *Xho*l site of the plCatH vector as described in US7968691. The LAT expression cassette (Xhol fragment) is shown in SEQ ID NO: 1.

The LAT precursor gene is shown *in italics* while the LAT 5'UTR is shown in bold (SEQ ID NO: 1).

CTCGAGGCTTTTCTTTTGGAAGAAAATATAGGGAAAATGGTACTTGTTAAAAATTCGGAATATTTATA
CAATATCATATGTTTCACATTGAAAGGGGAGGAGAATCATGAAACAACAAAAACGGCTTTACGCCCGA
TTGCTGACGCTGTTATTTGCGCTCATCTTCTTGCTGCCTCATTCTGCAGCTTCAGCAGCAAATCTTAA
TGGGACGCTGATGCAGTATTTTGAATGGTACATGCCCAATGACGGCCAACATTGGAAGCGTTTGCAAA
ACGACTCGGCATATTTGGCTGAACACGGTATTACTGCCGTCTGGATTCCCCCGGCATATAAGGGAACG
AGCCAAGCGGATGTGGGCTACGGTGCTTACGACCTTTATGATTTAGGGGAGTTTCATCAAAAAGGGAC
GGTTCGGACAAAGTACGGCACAAAAGGAGAGCTGCAATCTGCGATCAAAAGTCTTCATTCCCGCGACA
TTAACGTTTACGGGGATGTGGTCATCAACCACAAAGGCGGCGCTGATGCGACCGAAGATGTAACCGCG

GTTGAAGTCGATCCCGCTGACCGCAACCGCGTAATTTCAGGAGAACACCTAATTAAAGCCTGGACACA
TTTTCATTTTCCGGGGCGCGGCAGCACATACAGCGATTTTAAATGGCATTGGTACCATTTTGACGGAA
CCGATTGGGACGAGTCCCGAAAGCTGAACCGCATCTATAAGTTTCAAGGAAAGGCTTGGGATTGGGAA
GTTTCCAATGAAAACGGCAACTATGATTATTTGATGTATGCCGACATCGATTATGACCATCCTGATGT
CGCAGCAGAAATTAAGAGATGGGGCACTTGGTATGCCAATGAACTGCAATTGGACGGTTTCCGTCTTG
ATGCTGTCAAACACATTAAATTTTCTTTTTTGCGGGATTGGGTTAATCATGTCAGGGAAAAAACGGGG
AAGGAAATGTTTACGGTAGCTGAATATTGGCAGAATGACTTGGGCGCGCTGGAAAACTATTTGAACAA
AACAAATTTTAATCATTCAGTGTTTGACGTGCCGCTTCATTATCAGTTCCATGCTGCATCGACACAGG
GAGGCGGCTATGATATGAGGAAATTGCTGAACGGTACGGTCGTTTCCAAGCATCCGTTGAAATCGGTT
ACATTTGTCGATAACCATGATACACAGCCGGGGCAATCGCTTGAGTCGACTGTCCAAACATGGTTTAA
GCCGCTTGCTTACGCTTTTATTCTCACAAGGGAATCTGGATACCCTCAGGTTTTCTACGGGGATATGT
ACGGGACGAAAGGAGACTCCCAGCGCGAAATTCCTGCCTTGAAACACAAAATTGAACCGATCTTAAAA
GCGAGAAAACAGTATGCGTACGGAGCACAGCATGATTATTTCGACCACCATGACATTGTCGGCTGGAC
AAGGGAAGGCGACAGCTCGGTTGCAAATTCAGGTTTGGCGGCATTAATAACAGACGGACCCGGTGGGG
CAAAGCGAATGTATGTCGGCCGGCAAAACGCCGGTGAGACATGGCATGACATTACCGGAAACCGTTCG
GAGCCGGTTGTCATCAATTCGGAAGGCTGGGGAGAGTTTCACGTAAACGGCGGGTCGGTTTCAATTTA
TGTTCAAAGATGAGTTAACAGAGGACGGATTTCCTGAAGGAAATCCGTTTTTTTATTTTAAGCTTGGA
GACAAGGTAAAGGATAAAACCTCGAG

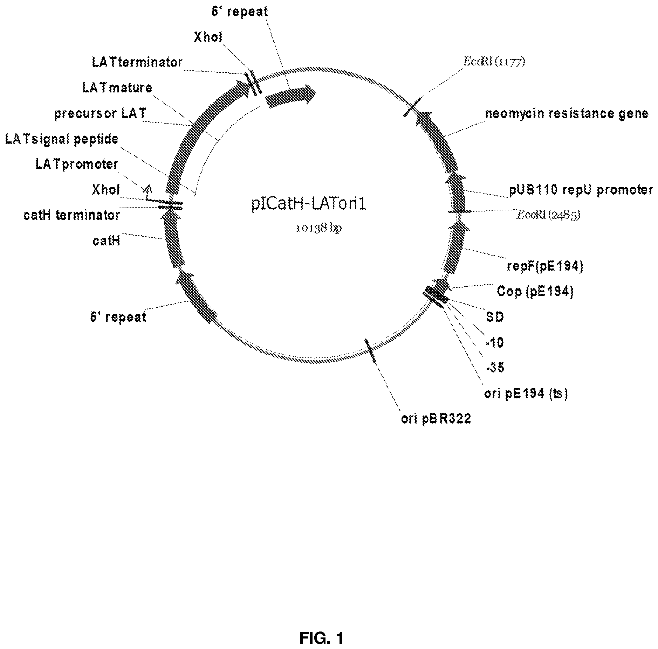

The plCatH-LATori1 plasmid (see, FIG. 1) was transformed into the genome of *B*. *licheniformis* strain BML780 as described in US Patent No. 7,968,691. After excision, amplification of the expression cassette up to 50 ppm chloramphenicol proved to be optimal for LAT production in the LAT 5'UTR strain.

By fusion PCR techniques, the LAT 5'UTR in plCatH-LATori1 was replaced by the *Bacillus subtilis aprE* 5'UTR. The nucleotide sequence of the *B*. *subtilis aprE* 5'UTR with an additional guanine at the 5' end is set forth as SEQ ID: NO: 2.

### GACAGAATAGTCTTTTAAGTAAGTCTACTCTGAATTTTTTTAAAAGGAGAGGGTAAAGA

The LAT expression cassette with the *aprE* 5'UTR (*Xho*I - *Not*I fragment) is shown in SEQ ID NO: 3. The LAT precursor gene is shown *in italics*, while the *aprE*-5'UTR is shown in bold:

CTCGAGGCTTTTCTTTTGGAAGAAAATATAGGGAAAATGGTACTTGTTAAAAATTCGGAATATTTATA

CAATATCATAT

GACAGAATAGTCTTTTAAGTAAGTCTACTCTGAATTTTTTTAAAAGGAGAGGGTAAAGAATGAAACAA

CAAAAACGGCTTTACGCCCGATTGCTGACGCTGTTATTTGCGCTCATCTTCTTGCTGCCTCATTCTGC

AGCTTCAGCAGCAAATCTTAATGGGACGCTGATGCAGTATTTTGAATGGTACATGCCCAATGACGGCC

AACATTGGAAGCGTTTGCAAAACGACTCGGCATATTTGGCTGAACACGGTATTACTGCCGTCTGGATT

CCCCCGGCATATAAGGGAACGAGCCAAGCGGATGTGGGCTACGGTGCTTACGACCTTTATGATTTAGG

GGAGTTTCATCAAAAAGGGACGGTTCGGACAAAGTACGGCACAAAAGGAGAGCTGCAATCTGCGATCA
AAAGTCTTCATTCCCGCGACATTAACGTTTACGGGGATGTGGTCATCAACCACAAAGGCGGCGCTGAT
GCGACCGAAGATGTAACCGCGGTTGAAGTCGATCCCGCTGACCGCAACCGCGTAATTTCAGGAGAACA
CCTAATTAAAGCCTGGACACATTTTCATTTTCCGGGGCGCGGCAGCACATACAGCGATTTTAAATGGC
ATTGGTACCATTTTGACGGAACCGATTGGGACGAGTCCCGAAAGCTGAACCGCATCTATAAGTTTCAA
GGAAAGGCTTGGGATTGGGAAGTTTCCAATGAAAACGGCAACTATGATTATTTGATGTATGCCGACAT
CGATTATGACCATCCTGATGTCGCAGCAGAAATTAAGAGATGGGGCACTTGGTATGCCAATGAACTGC
AATTGGACGGTTTCCGTCTTGATGCTGTCAAACACATTAAATTTTCTTTTTTGCGGGATTGGGTTAAT
CATGTCAGGGAAAAAACGGGGAAGGAAATGTTTACGGTAGCTGAATATTGGCAGAATGACTTGGGCGC
GCTGGAAAACTATTTGAACAAAACAAATTTTAATCATTCAGTGTTTGACGTGCCGCTTCATTATCAGT
TCCATGCTGCATCGACACAGGGAGGCGGCTATGATATGAGGAAATTGCTGAACGGTACGGTCGTTTCC
AAGCATCCGTTGAAATCGGTTACATTTGTCGATAACCATGATACACAGCCGGGGCAATCGCTTGAGTC
GACTGTCCAAACATGGTTTAAGCCGCTTGCTTACGCTTTTATTCTCACAAGGGAATCTGGATACCCTC
AGGTTTTCTACGGGGATATGTACGGGACGAAAGGAGACTCCCAGCGCGAAATTCCTGCCTTGAAACAC
AAAATTGAACCGATCTTAAAAGCGAGAAAACAGTATGCGTACGGAGCACAGCATGATTATTTCGACCA
CCATGACATTGTCGGCTGGACAAGGGAAGGCGACAGCTCGGTTGCAAATTCAGGTTTGGCGGCATTAA
TAACAGACGGACCCGGTGGGGCAAAGCGAATGTATGTCGGCCGGCAAAACGCCGGTGAGACATGGCAT
GACATTACCGGAAACCGTTCGGAGCCGGTTGTCATCAATTCGGAAGGCTGGGGAGAGTTTCACGTAAA
CGGCGGGTCGGTTTCAATTTATGTTCAAAGATGAGTTAACAGAGGACGGATTTCCTGAAGGAAATCCG
TTTTTTTATTTTAAGCTTGGAGACAAGGTAAAGGATAAAACAGCTGCGGCCGC

The pICatH plasmid containing the LAT (*i.e.*, *B.* licheniformis AmyL) gene fused to the aprE-5'UTR was transformed and integrated into the genome of strain BML780 as described above. After excision, amplification up to 25 ppm chloramphenicol proved to be optimal for LAT production in the aprE-5'UTR strain.

Production of LAT by strain BML780-LAT-CAP50 (LAT 5'UTR) was compared to production of BML780-[aprE-5'UTR]-LAT-CAP25 (*aprE* 5'UTR) by growing both strains in production medium (per liter: 10 g starch, 10 g soytone, 10 g soy flour, 20 g glucose, 3.6 g urea, 0.75 g CaCl₂.2H₂O, 21.75 g K₂HPO₄, 17 g KH₂PO₄, MOPS buffer, micronutrients; pH 6.8). After 68h of growth in an Infors incubator (37 °C, 300 rpm), LAT activity in supernatant was determined with Megazyme's α-Amylase Assay Kit using Ceralpha as substrate, according to the instruction of the supplier (Megazyme International, Ireland). The relative numbers for activity, i.e. productivity, are shown in Table 3.

**Table 3**

| **Production of LAT in the LAT 5'UTR strain versus the *aprE* 5'UTR strain** | | |
|---|---|---|
| | LAT 5'UTR | *aprE* 5'UTR |
| LATα-amylase | 100 | 119 |

### Example 2

### Improved production of Pullulanase PULm104 by the aprE 5'UTR

PULm104 is the *Bacillus deramificans* pullulanase from which the N-terminal 104 amino acids have been deleted. Construction of *Bacillus licheniformis* strains producing PULm104 are described in US8354101. The best producing strain described in US8354101 was BML780-PULm104-Ori1-CAP75, which contains the *amyL* (LAT) 5'UTR.

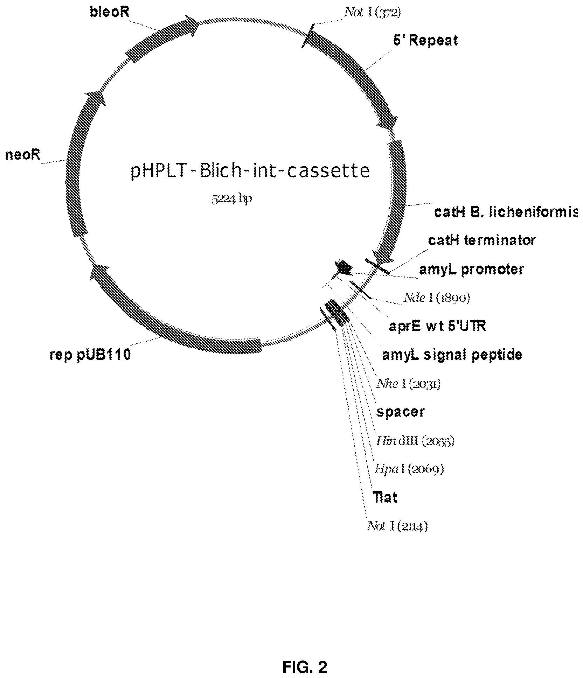

The *amyL* (LAT) 5'UTR was replaced by *aprE* 5'UTRs as outlined below. First, plasmid pHPLT-Blich-int-cassette was synthetically constructed by DNA2.0. Inc. (Menlo Park, CA, USA). The design of this plasmid was based on vector pHPLT (US5871550), vector pICatH (US8354101) and the *aprE* 5'UTR, UTR V2 or UTR V3 from *Bacillus subtilis* (Table below for sequences)). A map of vector pHPLT-Blich-int-cassette is shown in Figure 2.

The DNA sequence for *5'aprE* UTR-PULm104 expression cassette is shown below (the 5' UTR is underlined) (SEQ ID NO: 11):

CTCGAGGCTTTTCTTTTGGAAGAAAATATAGGGAAAATGGTACTTGTTAAAAATTCGGAATATTTATA
CAATATCATATGACAGAATAGTCTTTTAAGTAAGTCTACTCTGAATTTTTTTAAAAGGAGAGGGTAAA
GAATGAAACAACAAAAACGGCTTTACGCCCGATTGCTGACGCTGTTATTTGCGCTCATCTTCTTGCTG
CCTCATTCTGCAGCTTCAGCAGCTGCTAAACCGGCAGTCAGCAACGCTTATCTGGATGCCAGCAACCA
AGTCCTGGTCAAACTGAGCCAACCGCTGACACTTGGAGAAGGAGCGAGCGGATTTACGGTCCATGATG
ACACGGCGAACAAAGATATCCCGGTCACGAGCGTTAAAGATGCTAGCCTGGGCCAAGATGTCACAGCA
GTTCTGGCGGGCACGTTTCAACATATCTTTGGCGGATCAGATTGGGCACCGGATAATCACAGCACGCT
GCTGAAAAAAGTCACGAACAACCTGTATCAGTTTAGCGGAGATCTGCCGGAAGGCAACTATCAATATA
AAGTCGCCCTGAACGATAGCTGGAACAATCCGAGCTATCCGAGCGATAACATCAATCTGACAGTCCCG
GCAGGCGGAGCACATGTCACGTTTAGCTATATCCCGAGCACACATGCCGTCTATGACACGATCAACAA
CCCGAACGCCGATCTTCAAGTCGAAAGCGGCGTCAAAACGGATCTGGTCACAGTCACATTGGGAGAAG
ATCCGGATGTCAGCCATACACTGAGCATCCAAACGGATGGCTATCAAGCGAAACAAGTCATCCCGAGA
AACGTCCTGAACAGCAGCCAGTATTATTATAGCGGCGATGATCTGGGCAACACGTATACACAAAAAGC
GACGACGTTTAAAGTTTGGGCGCCGACAAGCACACAAGTCAACGTCCTGCTGTATGATTCAGCAACAG
GCAGCGTCACAAAAATCGTCCCGATGACAGCATCAGGACATGGAGTCTGGGAAGCGACGGTCAACCAA
AACCTGGAAAACTGGTATTATATGTATGAAGTCACGGGCCAAGGATCAACAAGAACAGCGGTCGATCC
GTATGCTACAGCAATCGCCCCGAATGGAACAAGAGGCATGATCGTCGATCTGGCAAAAACAGACCCGG
CAGGCTGGAATAGCGATAAACATATCACGCCGAAAAACATCGAAGATGAAGTCATCTATGAAATGGAC
GTCCGGGATTTTAGCATCGATCCGAACAGCGGCATGAAAAACAAAGGCAAATATCTGGCGCTGACGGA
AAAAGGAACAAAAGGCCCGGATAACGTCAAAACAGGCATCGATAGCCTGAAACAACTGGGCATCACAC
ATGTCCAACTGATGCCGGTCTTTGCTAGCAATAGCGTCGATGAAACGGACCCGACACAAGATAACTGG
GGCTATGACCCGAGAAATTATGATGTCCCGGAAGGCCAATATGCCACGAACGCCAATGGAAACGCCCG
GATCAAAGAATTTAAAGAAATGGTCCTGAGCCTTCATAGAGAACATATCGGCGTCAACATGGACGTCG
TCTATAACCATACGTTTGCCACACAGATCAGCGACTTTGATAAAATCGTGCCGGAATATTATTATCGG
ACGGATGACGCCGGCAATTATACGAATGGCAGCGGCACAGGAAATGAAATCGCCGCCGAAAGACCGAT

GGTCCAGAAATTTATCATCGACAGCCTTAAATATTGGGTCAACGAATATCATATCGACGGCTTTCGCT
TTGATCTGATGGCGCTGCTGGGCAAAGATACAATGAGCAAAGCGGCGAGCGAACTTCATGCTATCAAT
CCGGGCATCGCTCTTTATGGAGAACCGTGGACAGGAGGAACATCAGCACTGCCGGATGATCAACTGCT
GACAAAAGGCGCCCAAAAAGGAATGGGAGTCGCCGTCTTTAACGACAACCTGAGAAATGCCCTGGATG
GCAACGTTTTTGATAGCAGCGCCCAAGGATTTGCTACAGGAGCGACAGGACTGACAGATGCCATCAAA
AATGGCGTCGAAGGCAGCATCAACGATTTTACAAGCAGCCCGGGAGAGACGATCAATTATGTCACGAG
CCATGACAACTATACGCTGTGGGACAAAATCGCTCTGAGCAACCCGAATGATAGCGAAGCGGACCGGA
TCAAAATGGATGAACTGGCACAAGCAGTCGTCATGACATCACAAGGCGTCCCGTTTATGCAAGGCGGA
GAAGAAATGCTGAGAACGAAAGGCGGCAACGACAACAGCTATAATGCCGGCGATGCCGTCAATGAATT
TGACTGGAGCCGGAAAGCACAATATCCGGACGTCTTTAACTATTATTCAGGACTTATCCATCTGAGAC
TGGACCATCCGGCGTTTAGAATGACGACGGCGAACGAAATCAACAGCCATCTTCAGTTTCTGAACAGC
CCGGAAAATACGGTCGCCTATGAACTGACGGACCATGTGAACAAAGACAAATGGGGCAACATCATCGT
CGTTTATAACCCGAACAAAACGGTCGCCACAATCAATCTTCCGAGCGGCAAATGGGCAATCAATGCCA
CAAGCGGCAAAGTTGGAGAAAGCACACTGGGACAAGCAGAAGGATCAGTCCAAGTCCCGGGAATCAGC
ATGATGATCCTTCATCAAGAAGTCAGCCCGGACCACGGCAAAAAATAAGTTAACAGAGGACGGATTTC
CTGAAGGAAATCCGTTTTTTTATTTTAAGCTTGGAGACAAGGTAAAGGATAAAACAGCTGCGGCCGC
(SEQ ID NO: 11)

| Name | DNA Sequence |
|---|---|
| LAT 5' UTR (SEQ ID NO: 8) | GTTTCACATTGAAAGGGGAGGAGAAT |
| *AprE* 5' UTR WT with additional 5' guanine (SEQ ID NO: 2) | GACAGAATAGTCTTTTAAGTAAGTCTACTCTGAATTTTTTTAAAAGGAGAGGGTAAAGA |
| *AprE* 5' UTR V2 (SEQ ID NO: 9) | GACCGCATAGTCCGTTAAGTGGGTCTACGCGGAATTTTTTTAAAAGGAGAGGGTAAAGA |
| *AprE* 5' UTR V3 (SEQ ID NO: 10) | GACAGAATAGTCTTTTAAGTAAGTCTACTCTGTTTCACATTGAAAGGAAAGGAGAGGGTAATC |
| *AprE* 5' UTR WT (SEQ ID NO: 17) | ACAGAATAGTCTTTTAAGTAAGTCTACTCTGAATTTTTTTAAAAGGAGAGGGTAAAGA |

Next, a PCR was performed with primers PULm104-Vector-RV (CGGTTTAGCAGCTGCGCTAGCTGCAGAATGAGGC (SEQ ID NO: 4)) and PULm104-Vector-FW (CGGCAAAAAATGAAAGCTTCTCGAGGTTAACAGAGG (SEQ ID NO: 5)) using pHPLT-Blich-int-cassette (1 ng/µl) as template. A second PCR was performed with primers PulM104-Assbl.-FW (CAGCTAGCGCAGCTGCTAAACCGGCAGTCAGCAAC (SEQ ID NO: 6)) and PulM104-Assbl.-RV (CGAGAAGCTTTCATTTTTTGCCGTGGTCCGGGCTG (SEQ ID NO: 7)), using cell material of BML780-PULm104-Ori1-CAP75 as template. Both PCRs were performed according to NEB Q5 standard protocol, 26 cycles.

The PULm104 fragment was column purified and first completely digested with *Hin*dIII (NEB), and then partial digested by *Nhe*I (NEB). The vector fragment was also column purified and fully digested with *Nhe*I and *Hin*dIII. Both vector and a PULm104 digestion mixture were purified again and then ligated using Roche T4 rapid ligation kit (#11635379001). After RCA amplification (TempliPhi DNA Amplification Kit from GE Healthcare Life Sciences), RCA products were transformed into competent *B*. *subtilis* cells, plated on Heart Infusion agar (Difco) containing 5 ppm chloramphenicol and 10 ppm neomycin, and incubated overnight at 37°C. The next day, 24 cfu's from the transformation plate were replicated in duplicate on fresh Heart Infusion agar plates with 5 ppm chloramphenicol and 10 ppm neomycin, and incubated overnight. The next morning, the plate was covered with an overlay of 1% agar containing 0.1% AZCL-pullulan in 100 mM NaAc pH5. The plate was incubated for 3 hours at 37C and a halo positive (pullulanase positive) was picked from the replica plate, and grown in 10 ml TSB containing 5 ppm chloramphenicol and 10 ppm neomycin. 2 ml of the culture was used to prep plasmid DNA and 30 µl of the DNA prep was digested by *Not*I and Apal. A 4 Kb fragment containing the 5'repeat-catH-PULm104 expression cassette was purified from gel and self-ligated (T4 ligase Roche), RCA amplified and transformed into *Bacillus licheniformis* strain BML780. After selection on Heart Infusion agar containing 7.5 ppm of chloramphenicol, transformants were checked for pullulanase activity using the AZCL-pullulan overlay method, as described above. The sequence of the expression cassette in 4 pullulanase positive clones was confirmed by DNA sequencing (BaseClear, The Netherlands). The expression cassettes were amplified as described in US8354101. Amplification up to 25 ppm chloramphenicol proved to be optimal for pullulanase production in the *aprE* 5'UTR strains and the BML780-[aprE-5'UTR]-PULm104-Ori1-CAP25 strain was used in further studies.

Production of PULm104 by strain BML780-PULm104-Ori1-CAP75 (LAT 5'UTR) was compared to production of BML780-[aprE-5'UTR]-PULm104-Ori1-CAP25 (*aprE* 5'UTR) by growing both strains in production medium (see Example 1). After 68h of growth in an Infors incubation shaker (300 ppm, 37 °C), Pullulanase activity in supernatant was determined with Megazyme's Pullulanase assay using red-pullulan as substrate, according to the instruction of the supplier (Megazyme International, Ireland). The relative numbers for activity, i.e. productivity, are shown in Table 4. Version 2 and 3 of the 5' *aprE* UTR showed comparable protein production.

**Table 4**

| **Production of PULm104 in the LAT 5'UTR strain versus the *aprE* 5'UTR strain** | | |
|---|---|---|
| | LAT 5'UTR | *aprE* 5'UTR |
| PULm104 | 100 | 129 |

### Comparative Example 3

### Improved Production of a Protein of Interest Using aprE 5' UTR with Ribosomal RNA Promoters

An expression construct comprising an ribosomal RNA promoter (*e.g.*, P1 *rrnB,* P1 *rrnI,* P2 *rrnI,* P1 *rrnE,* P2 *rrnE* or P3 *rrnE*) or ribosomal protein promoter (*e.g.*, P*rpsD,* P1 *rpsJ,* P2 *rpsJ*) or sigma factor promoters ( e.g. P*rpoD*), *aprE* 5' UTR, a suitable signal sequence as described herein (*e.g.*, LAT, *aprE,* CBH1, Streptomyces cellulase gene signal sequence), a suitable gene of interest as described herein and a transcription terminator are cloned into the suitable restriction site (*e.g.*, Xhol) of a suitable vector (*e.g.*, pICatH). The expression cassettes are shown in SEQ ID NOs: 12 and 13.

The plasmid vectors containing the genes of interest are transformed and integrated into the genome of a suitable bacterial strain (*e.g.*, strain BML780 as described above).

Production of proteins of interest by the bacterial strains are compared to various control strains by growing the strains in production medium (*e.g.,* per liter: 10 g starch, 10 g soytone, 10 g soy flour, 20 g glucose, 3.6 g urea, 0.75 g CaCl₂.2H₂O, 21.75 g K₂HPO₄, 17 g KH₂PO₄, MOPS buffer, micronutrients; pH 6.8). After 68h of growth in an Infors incubator (37 °C, 300 rpm), the protein activity in supernatant is determined.

The *rrnl* P2 promoter (from *B. subtilis*) is shown as lowercase letters. The *aprE* 5'UTR is in **bold and underlined.** The mature coding chains are *in italics.* The STOP codon is **in bold.** The sequence holding the transcription terminator is underlined.
*rrnI* P2 promoter - *aprE* 5'UTR - LAT signal peptide - mature LAT expression construct is shown below (SEQ ID NO: 12): 
tcgctgataaacagctgacatcaactaaaagcttcattaaatactttgaaaaaagttgttgacttaaa
agaagctaaatgttatagtaataaaACAGAATAGTCTTTTAAGTAAGTCTACTCTGAATTTTTTTAAA
AGGAGAGGGTAAAGAATGAAACAACAAAAACGGCTTTACGCCCGATTGCTGACGCTGTTATTTGCGCT
CATCTTCTTGCTGCCTCATTCTGCAGCTAGCGCAGCAAATCTTAATGGGACGCTGATGCAGTATTTTG
AATGGTACATGCCCAATGACGGCCAACATTGGAAGCGTTTGCAAAACGACTCGGCATATTTGGCTGAA
CACGGTATTACTGCCGTCTGGATTCCCCCGGCATATAAGGGAACGAGCCAAGCGGATGTGGGCTACGG
TGCTTACGACCTTTATGATTTAGGGGAGTTTCATCAAAAAGGGACGGTTCGGACAAAGTACGGCACAA
AAGGAGAGCTGCAATCTGCGATCAAAAGTCTTCATTCCCGCGACATTAACGTTTACGGGGATGTGGTC
ATCAACCACAAAGGCGGCGCTGATGCGACCGAAGATGTAACCGCGGTTGAAGTCGATCCCGCTGACCG
CAACCGCGTAATTTCAGGAGAACACCTAATTAAAGCCTGGACACATTTTCATTTTCCGGGGCGCGGCA
GCACATACAGCGATTTTAAATGGCATTGGTACCATTTTGACGGAACCGATTGGGACGAGTCCCGAAAG
CTGAACCGCATCTATAAGTTTCAAGGAAAGGCTTGGGATTGGGAAGTTTCCAATGAAAACGGCAACTA
TGATTATTTGATGTATGCCGACATCGATTATGACCATCCTGATGTCGCAGCAGAAATTAAGAGATGGG
 
GCACTTGGTATGCCAATGAACTGCAATTGGACGGTTTCCGTCTTGATGCTGTCAAACACATTAAATTT
TCTTTTTTGCGGGATTGGGTTAATCATGTCAGGGAAAAAACGGGGAAGGAAATGTTTACGGTAGCTGA
ATATTGGCAGAATGACTTGGGCGCGCTGGAAAACTATTTGAACAAAACAAATTTTAATCATTCAGTGT
TTGACGTGCCGCTTCATTATCAGTTCCATGCTGCATCGACACAGGGAGGCGGCTATGATATGAGGAAA
TTGCTGAACGGTACGGTCGTTTCCAAGCATCCGTTGAAATCGGTTACATTTGTCGATAACCATGATAC
ACAGCCGGGGCAATCGCTTGAGTCGACTGTCCAAACATGGTTTAAGCCGCTTGCTTACGCTTTTATTC
TCACAAGGGAATCTGGATACCCTCAGGTTTTCTACGGGGATATGTACGGGACGAAAGGAGACTCCCAG
CGCGAAATTCCTGCCTTGAAACACAAAATTGAACCGATCTTAAAAGCGAGAAAACAGTATGCGTACGG
AGCACAGCATGATTATTTCGACCACCATGACATTGTCGGCTGGACAAGGGAAGGCGACAGCTCGGTTG
CAAATTCAGGTTTGGCGGCATTAATAACAGACGGACCCGGTGGGGCAAAGCGAATGTATGTCGGCCGG
CAAAACGCCGGTGAGACATGGCATGACATTACCGGAAACCGTTCGGAGCCGGTTGTCATCAATTCGGA
AGGCTGGGGAGAGTTTCACGTAAACGGCGGGTCGGTTTCAATTTATGTTCAAAGATGAGTTAACAGAG
GACGGATTTCCTGAAGGAAATCCGTTTTTTTATTTTAAGCTTGGAGACAAGGTAAAGGATAAAACAGC
TGCGGCCGC
*rrnl* P2 promoter- *aprE* 5'UTR - LAT signal peptide - mature PULm104 expression construct is shown below (SEQ ID NO: 13):

tcgctgataaacagctgacatcaactaaaagcttcattaaatactttgaaaaaagttgttgacttaaa
agaagctaaatgttatagtaataaaACAGAATAGTCTTTTAAGTAAGTCTACTCTGAATTTTTTTAAA
AGGAGAGGGTAAAGAATGAAACAACAAAAACGGCTTTACGCCCGATTGCTGACGCTGTTATTTGCGCT
CATCTTCTTGCTGCCTCATTCTGCAGCTAGCGCAGCTGCTAAACCGGCAGTCAGCAACGCTTATCTGG
ATGCCAGCAACCAAGTCCTGGTCAAACTGAGCCAACCGCTGACACTTGGAGAAGGAGCGAGCGGATTT
ACGGTCCATGATGACACGGCGAACAAAGATATCCCGGTCACGAGCGTTAAAGATGCTAGCCTGGGCCA
AGATGTCACAGCAGTTCTGGCGGGCACGTTTCAACATATCTTTGGCGGATCAGATTGGGCACCGGATA
ATCACAGCACGCTGCTGAAAAAAGTCACGAACAACCTGTATCAGTTTAGCGGAGATCTGCCGGAAGGC
AACTATCAATATAAAGTCGCCCTGAACGATAGCTGGAACAATCCGAGCTATCCGAGCGATAACATCAA
TCTGACAGTCCCGGCAGGCGGAGCACATGTCACGTTTAGCTATATCCCGAGCACACATGCCGTCTATG
ACACGATCAACAACCCGAACGCCGATCTTCAAGTCGAAAGCGGCGTCAAAACGGATCTGGTCACAGTC
ACATTGGGAGAAGATCCGGATGTCAGCCATACACTGAGCATCCAAACGGATGGCTATCAAGCGAAACA
AGTCATCCCGAGAAACGTCCTGAACAGCAGCCAGTATTATTATAGCGGCGATGATCTGGGCAACACGT
ATACACAAAAAGCGACGACGTTTAAAGTTTGGGCGCCGACAAGCACACAAGTCAACGTCCTGCTGTAT
GATTCAGCAACAGGCAGCGTCACAAAAATCGTCCCGATGACAGCATCAGGACATGGAGTCTGGGAAGC
GACGGTCAACCAAAACCTGGAAAACTGGTATTATATGTATGAAGTCACGGGCCAAGGATCAACAAGAA
CAGCGGTCGATCCGTATGCTACAGCAATCGCCCCGAATGGAACAAGAGGCATGATCGTCGATCTGGCA
AAAACAGACCCGGCAGGCTGGAATAGCGATAAACATATCACGCCGAAAAACATCGAAGATGAAGTCAT
CTATGAAATGGACGTCCGGGATTTTAGCATCGATCCGAACAGCGGCATGAAAAACAAAGGCAAATATC
TGGCGCTGACGGAAAAAGGAACAAAAGGCCCGGATAACGTCAAAACAGGCATCGATAGCCTGAAACAA

CTGGGCATCACACATGTCCAACTGATGCCGGTCTTTGCTAGCAATAGCGTCGATGAAACGGACCCGAC
ACAAGATAACTGGGGCTATGACCCGAGAAATTATGATGTCCCGGAAGGCCAATATGCCACGAACGCCA
ATGGAAACGCCCGGATCAAAGAATTTAAAGAAATGGTCCTGAGCCTTCATAGAGAACATATCGGCGTC
AACATGGACGTCGTCTATAACCATACGTTTGCCACACAGATCAGCGACTTTGATAAAATCGTGCCGGA
ATATTATTATCGGACGGATGACGCCGGCAATTATACGAATGGCAGCGGCACAGGAAATGAAATCGCCG
CCGAAAGACCGATGGTCCAGAAATTTATCATCGACAGCCTTAAATATTGGGTCAACGAATATCATATC
GACGGCTTTCGCTTTGATCTGATGGCGCTGCTGGGCAAAGATACAATGAGCAAAGCGGCGAGCGAACT
TCATGCTATCAATCCGGGCATCGCTCTTTATGGAGAACCGTGGACAGGAGGAACATCAGCACTGCCGG
ATGATCAACTGCTGACAAAAGGCGCCCAAAAAGGAATGGGAGTCGCCGTCTTTAACGACAACCTGAGA
AATGCCCTGGATGGCAACGTTTTTGATAGCAGCGCCCAAGGATTTGCTACAGGAGCGACAGGACTGAC
AGATGCCATCAAAAATGGCGTCGAAGGCAGCATCAACGATTTTACAAGCAGCCCGGGAGAGACGATCA
ATTATGTCACGAGCCATGACAACTATACGCTGTGGGACAAAATCGCTCTGAGCAACCCGAATGATAGC
GAAGCGGACCGGATCAAAATGGATGAACTGGCACAAGCAGTCGTCATGACATCACAAGGCGTCCCGTT
TATGCAAGGCGGAGAAGAAATGCTGAGAACGAAAGGCGGCAACGACAACAGCTATAATGCCGGCGATG
CCGTCAATGAATTTGACTGGAGCCGGAAAGCACAATATCCGGACGTCTTTAACTATTATTCAGGACTT
ATCCATCTGAGACTGGACCATCCGGCGTTTAGAATGACGACGGCGAACGAAATCAACAGCCATCTTCA
GTTTCTGAACAGCCCGGAAAATACGGTCGCCTATGAACTGACGGACCATGTGAACAAAGACAAATGGG
GCAACATCATCGTCGTTTATAACCCGAACAAAACGGTCGCCACAATCAATCTTCCGAGCGGCAAATGG
GCAATCAATGCCACAAGCGGCAAAGTTGGAGAAAGCACACTGGGACAAGCAGAAGGATCAGTCCAAGT
CCCGGGAATCAGCATGATGATCCTTCATCAAGAAGTCAGCCCGGACCACGGCAAAAAATAAGTTAACA
GAGGACGGATTTCCTGAAGGAAATCCGTTTTTTTATTTTAAGCTTGGAGACAAGGTAAAGGATAAAAC
AGCTGCGGCCGCTGAGTTAACAGAGGACGGATTTCCTGAAGGAAATCCGTTTTTTTATTTTAAGCTTG
GAGACAAGGTAAAGGATAAAACAGCTGCGGCCGC

### Example 4

### Improved Production of G. stearothermophilus Amylase Variant Using aprE 5' UTR and Variants Thereof

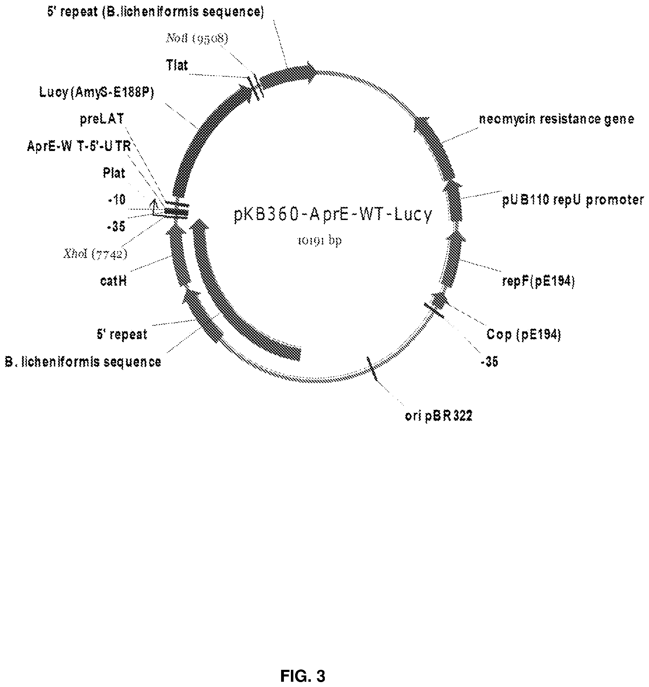

In the standard *B. licheniformis* integration vector, pKB360-CatH containing a G. *stearothermophilus* (AmyS) variant gene downstream of the LAT (*Bacillus licheniformis* AmyL) promoter, the LAT 5'-UTR sequence was replaced by AprE-WT 5'-UTR and AprE-V2-5'-UTR or AprE-V3-5'-UTR (see FIG. 3 for the generic plasmid map). The sequences for the expression cassettes are shown below.

The LAT promoter is in lowercase letters; the *aprE* 5'UTR is in **bold and underlined**; the LAT signal peptide is in lowercase letter and underlined; the mature chain of AmyS variant is *in italics*; the STOP codon is **in bold**; the sequence holding the transcription terminator is underlined.
LAT promoter - *aprE* 5'UTR - LAT signal peptide - mature AmyS variant (SEQ ID NO: 14): 
gcttttcttttggaagaaaatatagggaaaatggtacttgttaaaaattcggaatatttatacaatat
catatGACAGAATAGTCTTTTAAGTAAGTCTACTCTGAATTTTTTTAAAAGGAGAGGGTAAAGAatga
aacaacaaaaacggctttacgcccgattgctgacgctgttatttgcgctcatcttcttgctgcctcat
tctgcaGCTTCAGCAGCCGCACCGTTTAACGGTACCATGATGCAGTATTTTGAATGGTACTTGCCGGA
TGATGGCACGTTATGGACCAAAGTGGCCAATGAAGCCAACAACTTATCCAGCCTTGGCATCACCGCTC
TTTGGCTGCCGCCCGCTTACAAAGGAACAAGCCGCAGCGACGTAGGGTACGGAGTATACGACTTGTAT
GACCTCGGCGAATTCAATCAAAAAGGGACCGTCCGCACAAAATATGGAACAAAAGCTCAATATCTTCA
AGCCATTCAAGCCGCCCACGCCGCTGGAATGCAAGTGTACGCCGATGTCGTGTTCGACCATAAAGGCG
GCGCTGACGGCACGGAATGGGTGGACGCCGTCGAAGTCAATCCGTCCGACCGCAACCAAGAAATCTCG
GGCACCTATCAAATCCAAGCATGGACGAAATTTGATTTTCCCGGGCGGGGCAACACCTACTCCAGCTT
TAAGTGGCGCTGGTACCATTTTGACGGCGTTGATTGGGACGAAAGCCGAAAATTAAGCCGCATTTACA
AATTCAGGGGCATCGGCAAAGCGTGGGATTGGCCGGTAGACACAGAAAACGGAAACTATGACTACTTA
ATGTATGCCGACCTTGATATGGATCATCCCGAAGTCGTGACCGAGCTGAAAAACTGGGGGAAATGGTA
TGTCAACACAACGAACATTGATGGGTTCCGGCTTGATGCCGTCAAGCATATTAAGTTCAGTTTTTTTC
CTGATTGGTTGTCGTATGTGCGTTCTCAGACTGGCAAGCCGCTATTTACCGTCGGGGAATATTGGAGC
TATGACATCAACAAGTTGCACAATTACATTACGAAAACAAACGGAACGATGTCTTTGTTTGATGCCCC
GTTACACAACAAATTTTATACCGCTTCCAAATCAGGGGGCGCATTTGATATGCGCACGTTAATGACCA
ATACTCTCATGAAAGATCAACCGACATTGGCCGTCACCTTCGTTGATAATCATGACACCGAACCCGGC
CAAGCGCTTCAGTCATGGGTCGACCCATGGTTCAAACCGTTGGCTTACGCCTTTATTCTAACTCGGCA
GGAAGGATACCCGTGCGTCTTTTATGGTGACTATTATGGCATTCCACAATATAACATTCCTTCGCTGA
AAAGCAAAATCGATCCGCTCCTCATCGCGCGCAGGGATTATGCTTACGGAACGCAACATGATTATCTT
GATCACTCCGACATCATCGGGTGGACAAGGGAAGGGGTCACTGAAAAACCAGGATCCGGGCTGGCCGC
ACTGATCACCGATGGGCCGGGAGGAAGCAAATGGATGTACGTTGGCAAACAACACGCTGGAAAAGTGT
TCTATGACCTTACCGGCAACCGGAGTGACACCGTCACCATCAACAGTGATGGATGGGGGGAATTCAAA
GTCAATGGCGGTTCGGTTTCGGTTTGGGTTCCTAGAAAAACGACCTGAGTTAACAGAGGACGGATTTC
CTGAAGGAAATCCGTTTTTTTATTTT
LAT promoter - V2_5'UTR - LAT signal peptide - mature AmyS variant (SEQ ID NO: 15): 
gcttttcttttggaagaaaatatagggaaaatggtacttgttaaaaattcggaatatttatacaatat
catatGACCGCATAGTCCGTTAAGTGGGTCTACGCGGAATTTTTTTAAAAGGAGAGGGTAAAGAatga
aacaacaaaaacggctttacgcccgattgctgacgctgttatttgcgctcatcttcttgctgcctcat
tctgcaGCTTCAGCAGCCGCACCGTTTAACGGTACCATGATGCAGTATTTTGAATGGTACTTGCCGGA
TGATGGCACGTTATGGACCAAAGTGGCCAATGAAGCCAACAACTTATCCAGCCTTGGCATCACCGCTC
TTTGGCTGCCGCCCGCTTACAAAGGAACAAGCCGCAGCGACGTAGGGTACGGAGTATACGACTTGTAT
GACCTCGGCGAATTCAATCAAAAAGGGACCGTCCGCACAAAATATGGAACAAAAGCTCAATATCTTCA
AGCCATTCAAGCCGCCCACGCCGCTGGAATGCAAGTGTACGCCGATGTCGTGTTCGACCATAAAGGCG
 
GCGCTGACGGCACGGAATGGGTGGACGCCGTCGAAGTCAATCCGTCCGACCGCAACCAAGAAATCTCG
GGCACCTATCAAATCCAAGCATGGACGAAATTTGATTTTCCCGGGCGGGGCAACACCTACTCCAGCTT
TAAGTGGCGCTGGTACCATTTTGACGGCGTTGATTGGGACGAAAGCCGAAAATTAAGCCGCATTTACA
AATTCAGGGGCATCGGCAAAGCGTGGGATTGGCCGGTAGACACAGAAAACGGAAACTATGACTACTTA
ATGTATGCCGACCTTGATATGGATCATCCCGAAGTCGTGACCGAGCTGAAAAACTGGGGGAAATGGTA
TGTCAACACAACGAACATTGATGGGTTCCGGCTTGATGCCGTCAAGCATATTAAGTTCAGTTTTTTTC
CTGATTGGTTGTCGTATGTGCGTTCTCAGACTGGCAAGCCGCTATTTACCGTCGGGGAATATTGGAGC
TATGACATCAACAAGTTGCACAATTACATTACGAAAACAAACGGAACGATGTCTTTGTTTGATGCCCC
GTTACACAACAAATTTTATACCGCTTCCAAATCAGGGGGCGCATTTGATATGCGCACGTTAATGACCA
ATACTCTCATGAAAGATCAACCGACATTGGCCGTCACCTTCGTTGATAATCATGACACCGAACCCGGC
CAAGCGCTTCAGTCATGGGTCGACCCATGGTTCAAACCGTTGGCTTACGCCTTTATTCTAACTCGGCA
GGAAGGATACCCGTGCGTCTTTTATGGTGACTATTATGGCATTCCACAATATAACATTCCTTCGCTGA
AAAGCAAAATCGATCCGCTCCTCATCGCGCGCAGGGATTATGCTTACGGAACGCAACATGATTATCTT
GATCACTCCGACATCATCGGGTGGACAAGGGAAGGGGTCACTGAAAAACCAGGATCCGGGCTGGCCGC
ACTGATCACCGATGGGCCGGGAGGAAGCAAATGGATGTACGTTGGCAAACAACACGCTGGAAAAGTGT
TCTATGACCTTACCGGCAACCGGAGTGACACCGTCACCATCAACAGTGATGGATGGGGGGAATTCAAA
GTCAATGGCGGTTCGGTTTCGGTTTGGGTTCCTAGAAAAACGACCTGAGTTAACAGAGGACGGATTTC
CTGAAGGAAATCCGTTTTTTTATTTT
LAT promoter - V3_5'UTR - LAT signal peptide - mature AmyS variant (SEQ ID NO: 16)

gcttttcttttggaagaaaatatagggaaaatggtacttgttaaaaattcggaatatttatacaatat
catatGACAGAATAGTCTTTTAAGTAAGTCTACTCTGTTTCACATTGAAAGGAAAGGAGAGGGTAATC
atgaaacaacaaaaacggctttacgcccgattgctgacgctgttatttgcgctcatcttcttgctgcc
tcattctgcaGCTTCAGCAGCCGCACCGTTTAACGGTACCATGATGCAGTATTTTGAATGGTACTTGC
CGGATGATGGCACGTTATGGACCAAAGTGGCCAATGAAGCCAACAACTTATCCAGCCTTGGCATCACC
GCTCTTTGGCTGCCGCCCGCTTACAAAGGAACAAGCCGCAGCGACGTAGGGTACGGAGTATACGACTT
GTATGACCTCGGCGAATTCAATCAAAAAGGGACCGTCCGCACAAAATATGGAACAAAAGCTCAATATC
TTCAAGCCATTCAAGCCGCCCACGCCGCTGGAATGCAAGTGTACGCCGATGTCGTGTTCGACCATAAA
GGCGGCGCTGACGGCACGGAATGGGTGGACGCCGTCGAAGTCAATCCGTCCGACCGCAACCAAGAAAT
CTCGGGCACCTATCAAATCCAAGCATGGACGAAATTTGATTTTCCCGGGCGGGGCAACACCTACTCCA
GCTTTAAGTGGCGCTGGTACCATTTTGACGGCGTTGATTGGGACGAAAGCCGAAAATTAAGCCGCATT
TACAAATTCAGGGGCATCGGCAAAGCGTGGGATTGGCCGGTAGACACAGAAAACGGAAACTATGACTA
CTTAATGTATGCCGACCTTGATATGGATCATCCCGAAGTCGTGACCGAGCTGAAAAACTGGGGGAAAT
GGTATGTCAACACAACGAACATTGATGGGTTCCGGCTTGATGCCGTCAAGCATATTAAGTTCAGTTTT
TTTCCTGATTGGTTGTCGTATGTGCGTTCTCAGACTGGCAAGCCGCTATTTACCGTCGGGGAATATTG
GAGCTATGACATCAACAAGTTGCACAATTACATTACGAAAACAAACGGAACGATGTCTTTGTTTGATG
CCCCGTTACACAACAAATTTTATACCGCTTCCAAATCAGGGGGCGCATTTGATATGCGCACGTTAATG

ACCAATACTCTCATGAAAGATCAACCGACATTGGCCGTCACCTTCGTTGATAATCATGACACCGAACC
CGGCCAAGCGCTTCAGTCATGGGTCGACCCATGGTTCAAACCGTTGGCTTACGCCTTTATTCTAACTC
GGCAGGAAGGATACCCGTGCGTCTTTTATGGTGACTATTATGGCATTCCACAATATAACATTCCTTCG
CTGAAAAGCAAAATCGATCCGCTCCTCATCGCGCGCAGGGATTATGCTTACGGAACGCAACATGATTA
TCTTGATCACTCCGACATCATCGGGTGGACAAGGGAAGGGGTCACTGAAAAACCAGGATCCGGGCTGG
CCGCACTGATCACCGATGGGCCGGGAGGAAGCAAATGGATGTACGTTGGCAAACAACACGCTGGAAAA
GTGTTCTATGACCTTACCGGCAACCGGAGTGACACCGTCACCATCAACAGTGATGGATGGGGGGAATT
CAAAGTCAATGGCGGTTCGGTTTCGGTTTGGGTTCCTAGAAAAACGACCTGAGTTAACAGAGGACGGA
TTTCCTGAAGGAAATCCGTTTTTTTATTTT

After transformation, plasmids were integrated into the *cat* locus on the *B*. *licheniformis* chromosome. Subsequently, 'loop-out' strains were obtained through excision of vector sequences, leaving only the cat - LAT-5'-UTR-AmyS variant expression cassette integrated in the chromosome. The expression cassette was then amplified by subjecting the strains to a stepwise increase in chloramphenicol concentration (cap5, cap25, cap50, cap75 µg/ml). The secreted amylase activities were measured and are shown in Table 5.

**Table 5**

| Construct | Relative Secreted Amylase Activity (ppm) | | | | | | | |
|---|---|---|---|---|---|---|---|---|
| | 5 ppm cap | | 25 ppm cap | | 50 ppm cap | | 75 ppm cap | |
| LAT_5'-UTR | | 50 | | 100 | | 209 | | 210 |
| AprE-WT_5'_UTR | | 100 | | 235 | | 229 | | 232 |
| AprE-v2_5'-UTR | | 212 | | 209 | | 214 | | 208 |
| AprE-v3_5'-UTR | | 98 | | 111 | | 198 | | 199 |

Furthermore, the copy number of the expression cassette were measured after the amplification and compared to the amylase production and are summarized in Table 6.

**Table 6**

| **Relative Secreted Amylase Activity and Copy Number of the Expression Cassette** | | | |
|---|---|---|---|
| Construct | Amylase production | Copy number | Amplification rounds |
| LAT 5'- UTR | 100 | 14 | 4 |
| *aprE* 5'UTR | 115 | 4 | 2 |
| V2 | 100 | 3 | 1 |
| V3 | 90-110 | ND | 1 |

Thus, it can be seen that expression cassettes comprising an *aprE* 5' UTR or its variants require lower amounts of chloramphenicol concentrations to achieve comparable production and secretion of amylase compared to *LAT* 5' UTR. In other words, less amplification is required to achieve higher expression titers.

Although the foregoing compositions and methods have been described in some detail by way of illustration and example for purposes of clarity of understanding, it is readily apparent to those of ordinary skill in the art in light of the teachings herein that certain changes and modifications may be made thereto without departing from the scope of the appended claims.

Accordingly, the preceding merely illustrates the principles of the present compositions and methods. It will be appreciated that those skilled in the art will be able to devise various arrangements which, although not explicitly described or shown herein, embody the principles of the present compositions and methods and are included within its scope. Furthermore, all examples and conditional language recited herein are principally intended to aid the reader in understanding the principles of the present compositions and methods and the concepts contributed by the inventors to furthering the art, and are to be construed as being without limitation to such specifically recited examples and conditions. Moreover, all statements herein reciting principles, aspects, and embodiments of the present compositions and methods as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present compositions and methods, therefore, is not intended to be limited to the exemplary embodiments shown and described herein.

## Claims

1. A DNA molecule comprising, in a 5' to 3' direction, a 5' untranslated region (5' UTR) nucleic acid sequence operably linked to a nucleic acid sequence encoding a gene of interest (GOI), wherein the 5' UTR is derived from a *Bacillus* species (spp.) aprE protease gene, wherein the 5' UTR is heterologous to the GOI in which the 5' UTR is operably linked, and
wherein the 5' UTR comprises the sequence of SEQ ID NO: 9.

2. An expression cassette or vector comprising a DNA molecule of claim 1.

3. A host cell comprising an expression cassette or vector of claim 2, or a DNA molecule of claim 1.

4. A host cell according to claim 3, wherein the host cell is a *Bacillus* species, optionally wherein the *Bacillus* species is selected from the group consisting of *B. licheniformis, B. lentus, B. subtilis, B. amyloliquefaciens, B. brevis, B. stearothermophilus, B. al alophilus, B. coagulans, B. circulans, B. pumilus, B. lautus, B. clausii, B. megaterium,* and *B. thuringiensis.*

5. The DNA molecule of claim 1, wherein the gene of interest codes for an enzyme, optionally wherein the enzyme is an amylase or a pullulanase.

6. The DNA molecule of claim 5, wherein the gene of interest codes for a wild-type *Bacillus* spp. enzyme or a variant *Bacillus* spp. enzyme.

7. The DNA molecule of claim 5, wherein the enzyme is selected from the group consisting of acyl transferases, alpha-amylases, β-amylases, alpha-galactosidases, arabinosidases, aryl esterases, beta-galactosidases, carrageenases, catalases, cellobiohydrolases, cellulases, chondroitinases, cutinases, endo-beta-1 , 4- glucanases, endo-p-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, and xylosidases.

8. A DNA molecule comprising a sequence having 90% sequence identity to SEQ ID NO: 15 when determined using the Clustal alignment tool, wherein the DNA molecule comprises in the 5' to 3' direction: a LAT promoter in operable combination with an *aprE*-5'UTR in operable combination with a LAT signal peptide in operable combination with an open reading frame encoding a mature G. *stearothermophilus* variant amylase, wherein the *aprE-5'UTR* has the sequence of SEQ ID NO: 9, optionally wherein the DNA molecule comprises sequence SEQ ID NO: 15.

9. A method for increasing expression of a protein of interest in a *Bacillus* host cell comprising: growing a modified *Bacillus* host cell in a suitable medium to express the protein of interest; wherein the modified *Bacillus* host cell is transformed with a vector comprising an expression cassette comprising in the 5' to 3' direction, at least (i) an aprE-5' UTR derived from a *Bacillus* spp. and (ii) a nucleic acid sequence encoding a protein of interest; wherein the expression of the protein of interest is increased by at least 5% relative to the expression of the same protein of interest expressed from a parental *Bacillus* host cell which does not comprises an aprE-5' UTR operably linked to the nucleic acid sequence encoding the protein of interest, and wherein the 5' UTR comprises the sequence of SEQ ID NO: 9.

10. A method for increasing expression of a protein of interest in a modified *Bacillus* host cell using no more than 50 ppm of chloramphenicol for amplification, the method comprising: growing a modified *Bacillus* host cell in a suitable medium to express the protein of interest; wherein the modified *Bacillus* host cell is transformed with a vector comprising an expression cassette comprising in the 5' to 3' direction, (i) an aprE-5' UTR derived from a *Bacillus* spp. and (ii) a nucleic acid sequence encoding for the protein of interest; wherein the expression of the protein of interest is at least 5% relative to the expression of the same protein of interest expressed from a parental *Bacillus* host cell which does not comprises an aprE-5' UTR operably linked to the nucleic acid sequence encoding the protein of interest, and wherein the 5' UTR comprises the sequence of SEQ ID NO: 9.

11. The method of claim 9 or 10, wherein:
(a) the vector further comprises a promoter from a ribosomal RNA gene, optionally wherein the ribosomal RNA gene promoter is selected from the group consisting of P1 rmB, P1 rml, P2 rml, P1 rmE, P2 rrnE and P3 rrnE;
(b) the vector further comprises a promoter from a ribosomal protein gene, optionally wherein the ribosomal protein gene promoter is selected from the group consisting of PrpsD, P1 rpsJ, P2 rpsJ and PrpoD;
(c) the vector further comprises a promoter from a LAT (AmyL) gene; or
(d) the method further comprises recovering the protein of interest from the medium.

12. The method of claim 9 or 10, wherein:
(a) the protein of interest is secreted from the bacterium;
(b) the protein of interest accumulates within the bacterium;
(c) the protein of interest is a wild-type *Bacillus* spp. enzyme or a variant *Bacillus* spp. enzyme; or
(d) the protein of interest is an enzyme selected from the group consisting of acyl transferases, alpha-amylases, β-amylases, alpha- galactosidases, arabinosidases, aryl esterases, beta-galactosidases, carrageenases, catalases, cellobiohydrolases, cellulases, chondroitinases, cutinases, endo-beta-1 , 4- glucanases, endo-p-mannanases, esterases, exo-mannanases, galactanases, glucoamylases, hemicellulases, hyaluronidases, keratinases, laccases, lactases, ligninases, lipases, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, β-glucanases, tannases, transglutaminases, xylan acetyl-esterases, xylanases, xyloglucanases, and xylosidases, optionally wherein the enzyme is an amylase or a pullulanase.

## Patentansprüche

1. DNA-Molekül, umfassend, in einer Richtung von 5' zu 3', eine Nukleinsäuresequenz in einem nicht translatierten 5'-Bereich (5'-UTR), wirkverbunden mit einer Nukleinsäuresequenz, die für ein Gen von Interesse (GOI) kodiert, wobei der 5'-UTR abgeleitet ist von einem aprE-Proteasegen einer *Bacillus-Spezies* (spp.), wobei der 5'-UTR heterolog ist zu dem GOI, in dem der 5'-UTR wirkverbunden ist, und
wobei der 5'-UTR die Sequenz SEQ ID NO: 9 umfasst.

2. Expressionskassette oder Vektor, umfassend ein DNA-Molekül nach Anspruch 1.

3. Wirtszelle, umfassend eine Expressionskassette oder einen Vektor nach Anspruch 2, oder DNA-Molekül nach Anspruch 1.

4. Wirtszelle nach Anspruch 3, wobei die Wirtszelle eine *Bacillus-Spezies* ist, optional wobei die *Bacillus-*Spezies ausgewählt ist aus der Gruppe bestehend aus *B*. *licheniformis, B*. *lentus*, *B*. *subtilis, B*. *amyloliquefaciens, B*. *brevis, B*. *stearothermophilus, B*. *al alophilus, B*. *coagulans, B*. *circulans, B*. *pumilus, B*. *lautus, B*. *clausii, B*. *megaterium* und *B*. *thuringiensis.*

5. DNA-Molekül nach Anspruch 1, wobei das Gen von Interesse für ein Enzym kodiert, optional wobei das Enzym eine Amylase oder eine Pullulanase ist.

6. DNA-Molekül nach Anspruch 5, wobei das Gen von Interesse für ein Enzym eines *Bacillus* spp. vom Wildtyp oder für ein Enzym einer Varianten von *Bacillus* spp. kodiert.

7. DNA-Molekül nach Anspruch 5, wobei das Enzym ausgewählt ist aus der Gruppe bestehend aus Acyltransferasen, Alpha-Amylasen, β-Amylasen, Alpha-Galactosidasen, Arabinosidasen, Arylesterasen, Beta-Galactosidasen, Carrageenasen, Katalasen, Cellobiohydrolasen, Cellulasen, Chondroitinasen, Cutinasen, Endo-beta-1,4-Glucanasen, Endo-p-Mannanasen, Esterasen, Exo-Mannanasen, Galactanasen, Glucoamylasen, Hemicellulasen, Hyaluronidasen, Keratinasen, Laccasen, Lactasen, Ligninasen, Lipasen, Lipoxygenasen, Mannanasen, Oxidasen, Pektatlyasen, Pektinacetylesterasen, Pektinasen, Pentosanasen, Peroxidasen, Phenoloxidasen, Phosphatasen, Phospholipasen, Phytasen, Polygalacturonasen, Proteasen, Pullulanasen, Reduktasen, Rhamnogalacturonasen, β-Glucanasen, Tannasen, Transglutaminasen, Xylan-Acetylesterasen, Xylanasen, Xyloglucanasen und Xylosidasen.

8. DNA-Molekül, umfassend eine Sequenz, die bei Ermittlung unter Verwendung des Clustal-Alignment-Tools 90 % Sequenzidentität mit SEQ ID NO: 15 aufweist, wobei das DNA-Molekül in der Richtung von 5' nach 3' umfasst: einen LAT-Promotor in Wirkverbindung mit einem apriE-5'-UTR in Wirkverbindung mit einem LAT-Signalpeptid in Wirkverbindung mit einem offenen Leserahmen, der für eine reife Amylasevariante von G. *stearothermophilus* kodiert, wobei der apriE-5'-UTR die Sequenz von SEQ ID NO: 9 aufweist, optional wobei das DNA-Molekül die Sequenz SEQ ID NO: 15 umfasst.

9. Verfahren zum Steigern der Expression eines Proteins von Interesse in einer Bacillus-Wirtszelle, umfassend: Züchten einer modifizierten Bacillus-Wirtszelle in einem geeigneten Medium zum Exprimieren des Proteins von Interesse; wobei die modifizierte Bacillus-Wirtszelle mit einem Vektor transformiert wird, der eine Expressionskassette umfasst, umfassend, in Richtung von 5' nach 3' mindestens (i) einen von einer *Bacillus-*Spezies stammenden aprE-5'-UTR und (ii) eine Nukleinsäuresequenz, die für ein Protein von Interesse kodiert, umfasst; wobei die Expression des Proteins von Interesse um mindestens 5 % erhöht ist im Vergleich zur Expression des gleichen Proteins von Interesse, das von einer Vorläufer-Bacillus-Wirtszelle exprimiert wird, die keinen aprE-5'-UTR umfasst, der mit der Nukleinsäuresequenz wirkverbunden ist, die für das Protein von Interesse kodiert, und wobei der 5'-UTR die Sequenz von SEQ ID NO: 9 umfasst.

10. Ein Verfahren zum Steigern der Expression eines Proteins von Interesse in einer modifizierten *Bacillus-*Wirtszelle unter Verwendung von nicht mehr als 50 ppm Chloramphenicol zur Amplifikation, das Verfahren umfassend: Züchten einer modifizierten *Bacillus-*Wirtszelle in einem geeigneten Medium zum Exprimieren des Proteins von Interesse; wobei die modifizierte *Bacillus-*Wirtszelle mit einem Vektor transformiert wird, der eine Expressionskassette umfasst, umfassend, in Richtung von 5' nach 3' (i) einen von einer *Bacillus-Spezies* stammenden aprE-5'-UTR und (ii) eine Nukleinsäuresequenz, die für das Protein von Interesse kodiert, umfasst; wobei die Expression des Proteins von Interesse mindestens 5 % relativ zur Expression des gleichen Proteins von Interesse beträgt, das von einer Vorläufer-Bacillus-Wirtszelle exprimiert wird, die keinen aprE-5'-UTR umfasst, der mit der Nukleinsäuresequenz wirkverbunden ist, die für das Protein von Interesse kodiert, und wobei der 5'-UTR die Sequenz von SEQ ID NO: 9 umfasst.

11. Verfahren nach Anspruch 9 oder 10, wobei:
(a) der Vektor ferner einen Promotor von einem ribosomalen RNA-Gen umfasst, optional wobei der ribosomale RNA-Gen-Promotor ausgewählt ist aus der Gruppe bestehend aus P1 rrnB, P1 rrnl, P2 rrnl, P1 rrnE, P2 rrnE und P3 rrnE;
(b) der Vektor ferner einen Promotor von einem ribosomalen Proteingen umfasst, optional wobei der ribosomale Proteingen-Promotor ausgewählt ist aus der Gruppe bestehend aus PrpsD, P1 rpsJ, P2 rpsJ und PrpoD;
(c) der Vektor ferner einen Promotor von einem LAT(AmyL)-Gen umfasst; oder
(d) das Verfahren ferner was Wiedergewinnen des Proteins von Interesse aus dem Medium umfasst.

12. Verfahren nach Anspruch 9 oder 10, wobei:
(a) das Protein von Interesse von dem Bakterium sezerniert wird;
(a) das Protein von Interesse sich in dem Bakterium ansammelt;
(c) das Protein von Interesse ein Enzym eines *Bacillus* spp. vom Wildtyp oder ein Enzym einer Variante von *Bacillus* spp. ist; oder
(d) das Protein von Interesse ein Enzym ist, ausgewählt aus der Gruppe bestehend aus Acyltransferasen, Alpha-Amylasen, β-Amylasen, Alpha-Galactosidasen, Arabinosidasen, Arylesterasen, Beta-Galactosidasen, Carrageenasen, Katalasen, Cellobiohydrolasen, Cellulasen, Chondroitinasen, Cutinasen, Endo-beta-1,4-Glucanasen, Endo-p-Mannanasen, Esterasen, Exo-Mannanasen, Galactanasen, Glucoamylasen, Hemicellulasen, Hyaluronidasen, Keratinasen, Laccasen, Lactasen, Ligninasen, Lipasen, Lipoxygenasen, Mannanasen, Oxidasen, Pektatlyasen, Pektinacetylesterasen, Pektinasen, Pentosanasen, Peroxidasen, Phenoloxidasen, Phosphatasen, Phospholipasen, Phytasen, Polygalacturonasen, Proteasen, Pullulanasen, Reduktasen, Rhamnogalacturonasen, β-Glucanasen, Tannasen, Transglutaminasen, Xylan-Acetylesterasen, Xylanasen, Xyloglucanasen und Xylosidasen, optional wobei das Enzym eine Amylase oder eine Pullulanase ist.

## Revendications

1. Molécule d'ADN comprenant, dans un sens 5' à 3', une séquence d'acides nucléiques de région non traduite 5' (UTR 5') liée de manière fonctionnelle à une séquence d'acides nucléiques codant pour un gène d'intérêt (GOI), l'UTR 5' étant issu d'un gène de protéase aprE d'une espèce (spp.) de *Bacillus,* l'UTR 5' étant hétérologue au GOI dans lequel l'UTR 5' est lié de manière fonctionnelle, et
l'UTR 5'comprenant la séquence de SEQ ID NO: 9.

2. Cassette d'expression ou vecteur comprenant une molécule d'ADN selon la revendication 1.

3. Cellule hôte comprenant une cassette d'expression ou un vecteur selon la revendication 2, ou une molécule d'ADN selon la revendication 1.

4. Cellule hôte selon la revendication 3, la cellule hôte étant une espèce de *Bacillus,* l'espèce de *Bacillus* étant choisie dans le groupe constitué par *B. licheniformis, B. lentus, B. subtilis, B. amyloliquefaciens, B. brevis*, *B. stearothermophilus, B. al alophilus, B. coagulans, B. circulans, B. pumilus, B. lautus, B. clausii*, *B. megaterium*, et *B. thuringiensis.*

5. Molécule d'ADN selon la revendication 1, le gène d'intérêt codant pour une enzyme, éventuellement l'enzyme étant une amylase ou une pullulanase.

6. Molécule d'ADN selon la revendication 5, le gène d'intérêt codant pour une enzyme de *Bacillus* spp. de type sauvage ou un variant d'enzyme de *Bacillus* spp..

7. Molécule d'ADN selon la revendication 5, l'enzyme étant choisie dans le groupe constitué par des acyltransférases, des alpha-amylases, des β-amylases, des alpha-galactosidases, des arabinosidases, des aryl estérases, des bêta-galactosidases, des carraghénases, des catalases, des cellobiohydrolases, des cellulases, des chondroïtinases, des cutinases, des endo-bêta-1, des 4-glucanases, des endo-p-mannanases, des estérases, des exo-mannanases, des galactanases, des glucoamylases, des hémicellulases, des hyaluronidases, des kératinases, des laccases, des lactases, des ligninases, des lipases, des lipoxygénases, des mannanases, des oxydases, des pectate lyases, des pectine acétylestérases, des pectinases, des pentosanases, des peroxydases, des phénoloxydases, des phosphatases, des phospholipases, des phytases, des polygalacturonases, des protéases, des pullulanases, des réductases, des rhamnogalacturonases, des β-glucanases, des tannases, des transglutaminases, des xylanes acétyl-estérases, des xylanases, des xyloglucanases et des xylosidases.

8. Molécule d'ADN comprenant une séquence ayant 90 % d'identité de séquence avec la SEQ ID NO: 15 lorsqu'elle est déterminée en utilisant l'outil d'alignement Clustal, la molécule d'ADN comprenant dans le sens 5' à 3' : un promoteur LAT en combinaison fonctionnelle avec un UTR *aprE*-5' en combinaison fonctionnelle avec un peptide de signal LAT en combinaison fonctionnelle avec un cadre de lecture ouvert codant pour une amylase variante de *G. stearothermophilus* mature, *l'UTR aprE*-5' ayant la séquence de SEQ ID NO: 9, éventuellement la molécule d'ADN comprenant la séquence de SEQ ID NO: 15.

9. Procédé pour augmenter l'expression d'une protéine d'intérêt dans une cellule hôte de *Bacillus* comprenant : la culture d'une cellule hôte de *Bacillus* modifiée dans un milieu approprié pour exprimer la protéine d'intérêt ; la cellule hôte de *Bacillus* modifiée étant transformée par un vecteur comprenant une cassette d'expression comprenant dans le sens 5' à 3', au moins (i) un UTR aprE-5' issu d'une *Bacillus* spp. et (ii) une séquence d'acides nucléiques codant pour une protéine d'intérêt ; l'expression de la protéine d'intérêt étant augmentée d'au moins 5 % par rapport à l'expression de la même protéine d'intérêt exprimée à partir d'une cellule hôte de *Bacillus* parente qui ne comprend pas un UTR aprE-5' lié de manière fonctionnelle à la séquence d'acides nucléiques codant pour la protéine d'intérêt, et l'UTR 5'comprenant la séquence de SEQ ID NO: 9.

10. Procédé pour augmenter l'expression d'une protéine d'intérêt dans une cellule hôte de *Bacillus* modifiée n'utilisant pas plus de 50 ppm de chloramphénicol pour une amplification, le procédé comprenant : la culture d'une cellule hôte de *Bacillus* modifiée dans un milieu approprié pour exprimer la protéine d'intérêt ; la cellule hôte de *Bacillus* modifiée étant transformée par un vecteur comprenant une cassette d'expression comprenant dans le sens 5' à 3', au moins (i) un UTR aprE-5' issu d'une *Bacillus* spp. et (ii) une séquence d'acides nucléiques codant pour la protéine d'intérêt ; l'expression de la protéine d'intérêt étant augmentée d'au moins 5 % par rapport à l'expression de la même protéine d'intérêt exprimée à partir d'une cellule hôte de *Bacillus* parente qui ne comprend pas un UTR aprE-5' lié de manière fonctionnelle à la séquence d'acides nucléiques codant pour la protéine d'intérêt, et l'UTR 5'comprenant la séquence de SEQ ID NO: 9.

11. Procédé selon la revendication 9 ou 10,
(a) le vecteur comprenant en outre un promoteur provenant d'un gène d'ARN ribosomal, éventuellement le promoteur de gène d'ARN ribosomal étant choisi dans le groupe constitué par P1 rrnB, P1 rrnl, P2 rrnl, P1 rrnE, P2 rrnE et P3 rrnE ;
(b) le vecteur comprenant en outre un promoteur provenant d'un gène de protéine ribosomale, éventuellement le promoteur de gène de protéine ribosomale étant choisi dans le groupe constitué par PrpsD, P1 rpsJ, P2 rpsJ et PrpoD ;
(c) le vecteur comprenant en outre un promoteur provenant d'un gène de LAT (AmyL) ; ou
(d) le procédé comprenant en outre la récupération de la protéine d'intérêt depuis le milieu.

12. Procédé selon la revendication 9 ou 10,
(a) la protéine d'intérêt étant sécrétée par la bactérie ;
(b) la protéine d'intérêt s'accumulant à l'intérieur de la bactérie ;
(c) la protéine d'intérêt étant une enzyme de *Bacillus* spp. de type sauvage ou un variant d'enzyme de *Bacillus* spp. ; ou
(d) la protéine d'intérêt étant une enzyme choisie dans le groupe constitué par des acyltransférases, des alpha-amylases, des β-amylases, des alpha-galactosidases, des arabinosidases, des aryl estérases, des bêta-galactosidases, des carraghénases, des catalases, des cellobiohydrolases, des cellulases, des chondroïtinases, des cutinases, des endo-bêta-1, des 4-glucanases, des endo-p-mannanases, des estérases, des exo-mannanases, des galactanases, des glucoamylases, des hémicellulases, des hyaluronidases, des kératinases, des laccases, des lactases, des ligninases, des lipases, des lipoxygénases, des mannanases, des oxydases, des pectate lyases, des pectine acétylestérases, des pectinases, des pentosanases, des peroxydases, des phénoloxydases, des phosphatases, des phospholipases, des phytases, des polygalacturonases, des protéases, des pullulanases, des réductases, des rhamnogalacturonases, des β-glucanases, des tannases, des transglutaminases, des xylanes acétyl-estérases, des xylanases, des xyloglucanases et des xylosidases, éventuellement l'enzyme étant une amylase ou une pullulanase.
